# EUROPEAN PATENT APPLICATION

(11) **EP 1 995 252 A1**
(43) Date of publication of application: **26.11.2008**
(21) Application number: 07714924.3
(22) Date of filing: 26.02.2007
(51) Int. Cl.: C07H 19/067, C07H 19/10, C07H 19/167, C07H 19/20, C07H 21/02, C07H 21/04

(54) **METHOD FOR REMOVAL OF NUCLEIC ACID-PROTECTING GROUP**

(30) Priority: 27.02.2006 JP 2006050394
(71) Applicant: Nippon Shinyaku Co., Ltd., Kyoto-shi, Kyoto 601-8550 (JP)
(72) Inventor: KITAGAWA, Hidetoshi, Ibaraki 305-0034 (JP); MASUDA, Hirofumi, Joso-shi, Ibaraki 300-2742 (JP)
(74) Representative: Vossius & Partner
(86) International application number: PCT/JP2007/053492
(87) International publication number: WO 2007/097447

(57) **Abstract**

The main object of the present invention is to provide a method for efficiently removing the silicon substituent which protects the 3'-hydroxyl group and the 5'-hydroxyl group of a ribose of a ribonucleic acid derivative in which the 2'-hydroxyl group of the ribose is protected with the following substituent (I) and the 3'-hydroxyl group and the 5'-hydroxyl group of the ribose are protected with a silyl protecting group.

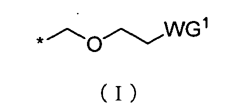

In the general formula (I), WG¹ represents an electron-withdrawing group.

A ribonucleic acid derivative represented by the following general formula (3) can be produced by allowing a salt of a tertiary amine with hydrofluoric acid represented by the following general formula (2) or a mixture of the tertiary amine and hydrofluoric acid to act on a ribonucleic acid derivative represented by the following general formula (1).

## Description

### FIELD OF THE INVENTION

The present invention relates to a method for removing silyl protecting groups for the 3'-hydroxyl group and the 5'-hydroxyl group of a ribose of a ribonucleic acid derivative in which the 2' -hydroxyl group of the ribose is protected with the following substituent (I) and the 3'-hydroxyl group and the 5'-hydroxyl group of the ribose are protected with a silyl protecting group. 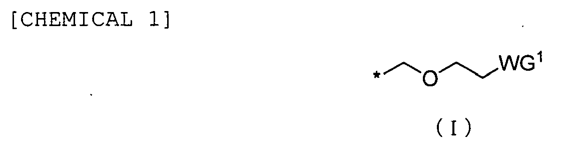 In the general formula (I), WG¹ represents an electron-withdrawing group.
Examples of the "electron-withdrawing group" related to the WG¹ may include cyano, nitro, alkylsulfonyl, arylsulfonyl and halogen. Among them, cyano is preferred.
Examples of the "alkyl" moiety of "alkylsulfonyl" related to the WG¹ may include straight or branched alkyl having 1 to 5 carbon atoms. Specifically, the alkyl may include, for example, methyl, ethyl, n-propyl, isopropyl, n-butyl, isobutyl, sec-butyl, tert-butyl, n-pentyl, isopentyl, neopentyl and tert-pentyl.
Examples of the "aryl" moiety of "arylsulfonyl" related to the WG¹ may include aryl groups having 6 to 12 carbon atoms. Specifically, the aryl may include, for example, phenyl, 1-naphthyl, 2-naphthyl and biphenyl. The aryl may be substituted, and examples of the "substituent" may include halogen, alkyl, alkoxy, cyano and nitro. The aryl may be substituted by 1 to 3 of these substituents at arbitrary positions.

### BACKGROUND ART

It is well known that an oligoribonucleic acid (oligo-RNA) is useful as an RNA probe for genetic analysis, a material for an RNA medicine (an antisense RNA, a ribozyme, or an RNA species that regulates gene expression through the RNAi effect), an artificial enzyme, or an aptamer.
As a reagent for producing an oligo-RNA, a phosphoramidite compound in which the 2'-hydroxyl group of a ribose is protected by substitution by the 2-cyanoethoxymethyl (CEM) group, which can be removed under neutral conditions, is known (Non-patent document 1). Further, Wada et al. also have provided as a reagent for producing an oligo-RNA, for example, a phosphoramidite compound in which the 1-(2-cyanoethoxy)ethyl (CEE) group is introduced at the 2'-hydroxyl position (Non-patent document 2 and Non-patent document 3).
In a process for producing such a phosphoramidite compound, Wada et al. have reported that in order to remove disiloxyl groups which protect the 3'-hydroxyl group and the 5'-hydroxyl group of a ribose, a fluorinating agent (tetrabutylammonium fluoride (hereinafter referred to as "TBAF"), triethylamine trihydrofluoride, pyridine hydrofluoride or the like) and an acid (acetic acid, hydrochloric acid, or sulfuric acid) can be used as a mixed reagent at an arbitrary mixing ratio (Patent document 1). However, only an example in which disiloxyl group is removed by using a mixed reagent of TBAF and acetic acid have been previously described.
Further, Saneyoshi et al. have reported that in a process for producing a phosphoramidite compound in which the 2'-hydroxyl group is substituted by 2-cyanoethyl, in order to remove the disiloxyl group which protects the 3'-hydroxyl group and the 5'-hydroxyl group of a ribose, a mixed reagent of triethylamine trihydrofluoride and triethylamine at a mixing ratio of 1:0.5 can be used (Non-patent document 4).
Patent document 1: WO 2005/023828 A1
Non-patent document 1: Ohgi et al., Organic Letters, Vol. 7, 3477 (2005)
Non-patent document 2: Takeshi Wada, Bio Industry, Vol. 21, No. 1, 17 (2004)
Non-patent document 3: T. Umemoto et al. , Tetrahedron Letters, Vol. 45, 9529 (2004)
Non-patent document 4: Hisao Saneyoshi et al., Journal of Organic Chemistry, 70, 10453 (2005)

### DISCLOSURE OF THE INVENTION

### PROBLEM TO BE SOLVED BY THE INVENTION

A main object of the present invention is to provide a method for efficiently removing a silicon substituent which protects the 3'-hydroxyl group and the 5'-hydroxyl group of a ribose of a ribonucleic acid derivative in which the 2'-hydroxyl group of the ribose is protected with the following substituent (I) and the 3'-hydroxyl group and the 5'-hydroxyl group of the ribose are protected with a silyl protecting group. 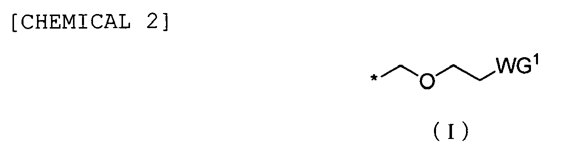 In the general formula (I), WG¹ has the same meanings as above.

### MEANS TO SOLVE THE PROBLEM

As a result of extensive studies for achieving the above object, the present inventors have found that a ribonucleic acid derivative represented by the following general formula (3) can be efficiently produced by allowing a salt of a tertiary amine with hydrofluoric acid represented by the following general formula (2) or a mixture of the tertiary amine and hydrofluoric acid to act on a ribonucleic acid derivative represented by the following general formula (1), and thus the present invention has been completed. 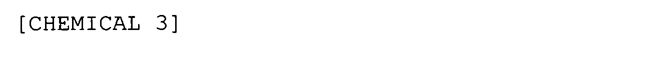 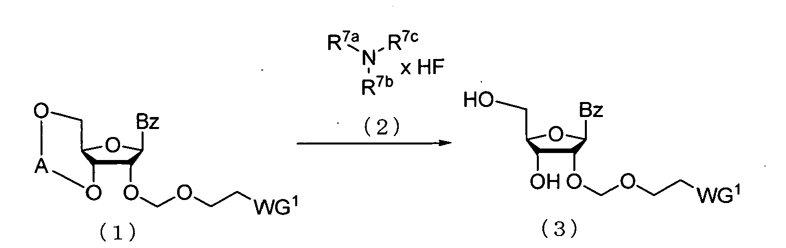 In the general formulae (1), (2) and (3), B_{Z} represents a nucleobase which may have protecting groups, or a modified form thereof. WG¹ has the same meanings as above. R^{7a}, R^{7b} and R^{7c} are the same or different from one another and represent alkyl; or R^{7a}, R^{7b} and R^{7c} represent a saturated bicyclic amino group when combined together with the adjacent nitrogen atom. x represents an integer in the range of 1 to 30. A represents a silicon substituent represented by the following general formulae (4a) and (4b). 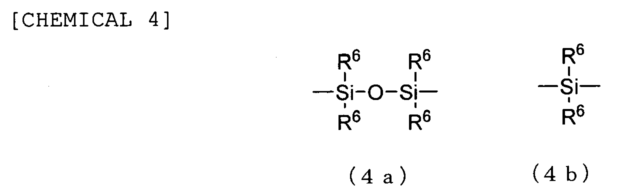 In the following general formula (4a) and (4b), R⁶ represents alkyl.

Examples of the "nucleobase" related to B_{Z} is not particularly limited as long as it is a nucleobase to be used in the synthesis of a nucleic acid, and examples thereof may include pyrimidine bases such as cytosine and uracil, and purine bases such as adenine and guanine. The "nucleobase" related to B_{Z} may be protected, and particularly in the case of a nucleobase having an amino group such as adenine, guanine or cytosine, the amino group thereof is preferably protected. The "protecting group for the amino group" is not particularly limited as long as it is a protecting group used as a protecting group of a nucleic acid, and specific examples thereof may include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. The "modified form" related to B_{Z} is the group in which a nucleobase has been substituted by an arbitrary substituent. Examples of the "substituent" related to the "modified form" of B_{Z} may include halogen, acyl, alkyl, arylalkyl, alkoxy, alkoxyalkyl, hydroxyl, amino, monoalkylamino, dialkylamino, carboxy, cyano, and nitro. The modified form related to B_{Z} may be substituted by 1 to 3 of these substituents at arbitrary positions.
Examples of the "halogen" related to the modified form of B_{Z} may include fluorine, chlorine, bromine and iodine.
Examples of the "acyl" related to the modified form of B_{Z} may include straight or branched alkanoyl having 1 to 6 carbon atoms and aroyl having 7 to 13 carbon atoms. Specifically, the acyl may include, for example, formyl, acetyl, n-propionyl, isopropionyl, n-butyryl, isobutyryl, tert-butyryl, valeryl, hexanoyl, benzoyl, naphthoyl, and levulinyl.
Examples of the "alkyl" related to the modified form of B_{Z} may include straight or branched alkyl having 1 to 5 carbon atoms. Specifically, the alkyl may include, for example, methyl, ethyl, n-propyl, isopropyl, n-butyl, isobutyl, sec-butyl, tert-butyl, n-pentyl, isopentyl, neopentyl, and tert-pentyl. The alkyl may be substituted and examples of the "substituent" may include halogen, alkyl, alkoxy, cyano and nitro. The alkyl may be substituted by 1 to 3 of these substituents at arbitrary positions. Examples of the "alkyl" moiety of the "arylalkyl", "alkoxyalkyl", "monoalkylamino", "dialkylamino" and "alkylsulfonyl" related to the modified form of B_{Z} may include the same ones as those illustrated for the "alkyl" mentioned above.
Examples of the "alkoxy" related to the modified form of B_{Z} may include straight or branched alkoxy having 1 to 4 carbon atoms. Specifically, the alkoxy may include, for example, methoxy, ethoxy, n-propoxy, isopropoxy, n-butoxy, isobutoxy, sec-butoxy and tert-butoxy. Among these, alkoxy groups having 1 to 3 carbon atoms are preferable, and methoxy is more preferable. Examples of the "alkoxy" moiety of the "alkoxyalkyl" related to the modified form of B_{Z} may include the same ones as those illustrated for the "alkoxy" mentioned above.
Examples of the "aryl" moiety of the "arylalkyl" related to the modified form of B_{Z} may include aryl groups having 6 to 12 carbon atoms. Specifically, the aryl may include, for example, phenyl, 1-naphthyl, 2-naphthyl and biphenyl. The aryl may be substituted, and examples of the "substituent" may include halogen, alkyl, alkoxy, cyano, and nitro. The aryl may be substituted by 1 to 3 of these substituents at arbitrary positions.
Examples of the "halogen", "alkyl" or "alkoxy", which are substituents of the alkyl or aryl related to the modified form of B_{Z}, may include the same ones as those illustrated in the above description, respectively.
Examples of the "alkyl" related to R⁶ may include straight or branched alkyl having 1 to 5 carbon atoms. Specifically, the alkyl may include, for example, methyl, ethyl, n-propyl, isopropyl, n-butyl, isobutyl, sec-butyl, tert-butyl, n-pentyl, isopentyl, neopentyl and tert-pentyl.
Examples of the salt of a tertiary amine with hydrofluoric acid or a mixture of a tertiary amine and hydrofluoric acid, which can be used in the present invention, may include a salt of a tertiary amine with hydrofluoric acid represented by the above-mentioned general formula (2) or a mixture obtained by mixing the tertiary amine and hydrofluoric acid in an appropriate solvent at an arbitrary ratio.
Examples of the "alkyl" related to R^{7a}, R^{7b} and R^{7c} may include the same ones as those illustrated for the "alkyl" related to the above-mentioned modified form of B_{Z}.
Examples of the "saturated bicyclic amino group" related to R^{7a}, R^{7b} and R^{7c} may include quinuclidine and triethylenediamine.
x represents a number in the range of 1 to 30 and may be a fractional number. x is preferably a number in the range of 2 to 15, and more preferably a number in the range of 3 to 10.
Examples of the "tertiary amine" to be used for the present invention may include trimethylamine, triethylamine, tripropylamine, tri-n-butylamine, N,N-diisopropylethylamine, quinuclidine, and triethylenediamine.
Examples of the "salt of a tertiary amine with hydrofluoric acid" according to the present invention may include trimethylamine trihydrofluoride, trimethylamine tetrahydrofluoride, trimethylamine pentahydrofluoride, trimethylamine hexahydrofluoride, trimethylamine pentahydrofluoride, triethylamine dihydrofluoride, triethylamine trihydrofluoride, triethylamine tetrahydrofluoride, triethylamine 26 hydrofluoride, quinuclidine trihydrofluoride and triethylenediamine tetrahydrofluoride (see, for example, Journal of Molecular Structure, 193, 247 (1989), Polish Journal of Chemistry, 67 (2), 281 (1993), Chemistry-A European Journal, 4 (6), 1043 (1998), Journal of Fluorine Chemistry, 118 (1-2), 123, (2002)). Among them, triethylamine trihydrofluoride is particularly preferred.
Further, examples of the "mixture of a tertiary amine and hydrofluoric acid" which can be used in the present invention may include a mixture obtained by mixing any of the above-mentioned tertiary amines and hydrofluoric acid in an appropriate solvent (for example, THF, acetonitrile, methanol, isopropanol, or toluene) at a mixing ratio (molar ratio) of, for example, 1:1 to 1:30 (tertiary amine : hydrofluoric acid). The mixing ratio (molar ratio) thereof is preferably from 1: 2 to 1: 15 (tertiary amine : hydrofluoric acid), and more preferably from 1:3 to 1:10 (tertiary amine : hydrofluoric acid).

Further, the present invention provides a method for producing a phosphoramidite compound represented by the following general formula (A) (hereinafter referred to as "phosphoramidite compound (A)") including the step of producing a ribonucleic acid derivative represented by the following general formula (3) by allowing a salt of a tertiary amine with hydrofluoric acid represented by the following general formula (2) or a mixture of the tertiary amine and hydrofluoric acid to act on a ribonucleic acid derivative represented by the following general formula (1) thereby to remove the silicon substituent which protects the 3'-hydroxyl group and the 5'-hydroxyl group of a ribose thereof. 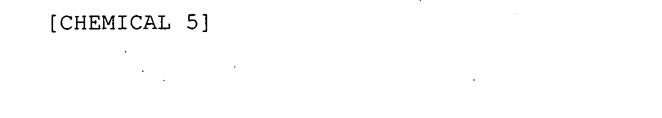 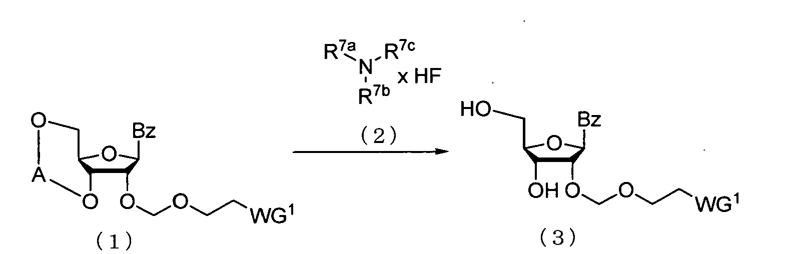 In the general formulae (1), (2) and (3), A, B_{Z}, R^{7a}, R^{7b}, R^{7c}, WG¹ and x have the same meanings as above. 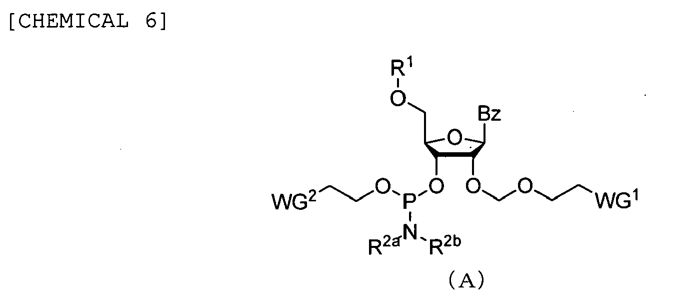 In the general formula (A), B_{Z} and WG¹ have the same meanings as above. R^{2a} and R^{2b} are the same or different and represent alkyl; or R^{2a} and R^{2b} represent a 5- or 6-membered saturated cyclic amino group when combined together with the adjacent nitrogen atom. The saturated cyclic amino group may have one oxygen atom or one sulfur atom as a ring-forming atom in addition to the nitrogen atom. WG² represents an electron-withdrawing group. R¹ represents a substituent represented by the following general formula (5). 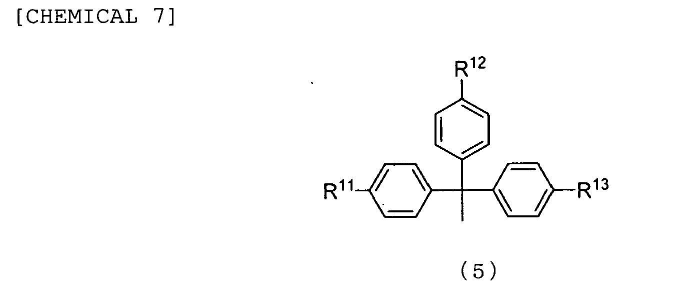 In the general formula (5), R¹¹, R¹² and R¹³ are the same or different and each represents hydrogen or alkoxy.
Examples of the "alkoxy" related to R¹¹, R¹² and R¹³ may include the same ones as those illustrated for the "alkoxy" related to the above-mentioned modified form of B_{Z}.
Examples of the "alkyl" related to R^{2a} and R^{2b} may include the same ones as those illustrated for the "alkyl" related to the above-mentioned modified form of B_{Z}.
Examples of the "5- or 6-membered saturated cyclic amino group" related to R^{2a} and R^{2b} may include pyrrolidin-1-yl, piperidin-1-yl, morpholin-1-yl and thiomorpholin-1-yl. Examples of the "electron-withdrawing group" related to the WG¹ may include the same ones as those illustrated for the "electron-withdrawing group" related to the above-mentioned WG².

The phosphoramidite compound (A) is a phosphoramidite compound having an ether-type protecting group represented by the below-mentioned general formula (I) at the 2'-hydroxy position, which can be removed under neutral conditions. In addition, the phosphoramidite compound (A) is characterized by the facts that the condensation reaction proceeds in a shorter time and results in a better yield during the synthesis of oligo-RNAs when compared with a conventional phosphoramidite compound. This is because the ether-type protecting group introduced at the 2'-hydroxyl position is a linear protecting group and therefore does not sterically crowd the space around the phosphorus atom attached to the 3'-hydroxyl group. The phosphoramidite compound (A) makes it possible to produce oligo-RNAs (A) of high purity by essentially the same method used in the production of oligo-DNAs. 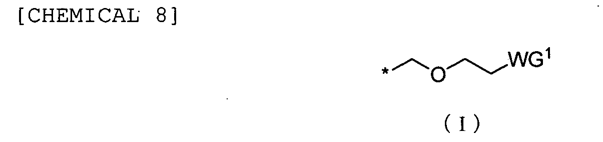 In the general formula (I), WG¹ has the same meanings as above.

In the present document, the term "oligo-DNA" means an oligonucleic acid having deoxyribonucleotides only. In addition, in the present document, the term "oligo-RNA" means an oligonucleic acid containing at least one ribonucleotide and which may also have one or more deoxyribonucleotides.

The present invention is explained in detail as follows.

### BEST MODE FOR CARRYING OUT THE INVENTION

In the following production method, it is common, when raw materials have a substituent that affects the reaction (e.g., hydroxyl, amino or carboxy), the reactions to be carried out after the raw materials have been protected with a suitable protecting group according to a known method. After the reaction has been completed, the protecting group can be removed by a known method such as catalytic reduction, alkali treatment, acid treatment or the like.

### I. Method of producing the phosphoramidite compound (A) according to the present invention

The phosphoramidite compound (A) can be produced as follows.
The phosphoramidite compounds (A) can be produced from a known compound or an intermediate which can easily be produced through the following Steps a to e, for example.
The method of producing the phosphoramidite compound (A) is described in detail below.

### (1) Step a:

Process for producing a ribonucleic acid compound represented by the following general formula (1), wherein an ether-type protecting group which can be removed under neutral conditions is introduced at the 2'-hydroxyl position, by allowing an alkylating reagent to act on a ribonucleic acid derivative represented by the following general formula (6). 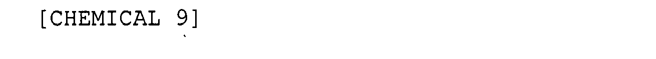 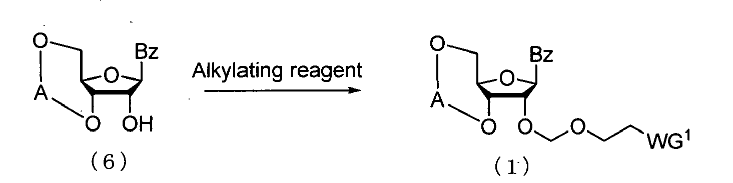 In the general formula (1) and (6), B_{Z}, A and WG¹ have the same meanings as above.
Examples of the "alkylating reagent" may include an ether compound represented by the following general formula (11). 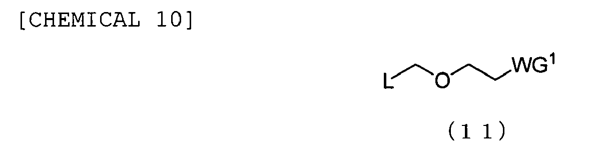 In the general formula (11), L represents halogen, an arylthio group, an alkylsulfoxide group or an alkylthio group. WG¹ has the same meanings as above.
Examples of the "halogen", the "aryl" moiety of the "arylthio group", the "alkyl" moiety of the "alkylsulfoxide group", the "alkylthio group" related to the L may include the same ones as those related to the above-mentioned modified form of B_{Z}, respectively.
Specific examples of the ether compound (11) may include the following compounds 1 and 2:
1. chloromethyl 2-cyanoethyl ether
2. 2-cyanoethyl methylthiomethyl ether
The ether compound (11) is a new alkylating reagent which can introduce an ether-type substituent, which is removable under neutral conditions, to the 2'-hydroxyl position under basic conditions, and which is useful as a reagent for producing the phosphoramidite compound (A).

The ether compounds (11) can be produced by the following Steps 1 to 4.

### Step 1:

Process for producing a compound represented by the following general formula (14) by alkylthiomethylating an alcohol compound represented by the following general formula (13). 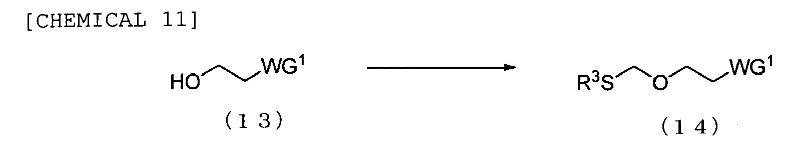 In the general formula (13) and (14), WG¹ has the same meanings as above. R³ represents alkyl or aryl.
The compound (14) is an ether compound (11), wherein L is an alkylthio group.
Examples of "alkyl" related to R³ may include the same ones as those illustrated for the "alkyl" related to the above-mentioned modified form of B_{Z}.
When R³ is methyl, examples of the "alkylthiomethylating reagent" may include a mixed solvent containing dimethylsulfoxide, acetic anhydride and acetic acid. The amount of dimethylsulfoxide to be used may be in the range of 10 to 200 mol per mol of the compound (13), and preferably 20 to 100 mol per mol of the compound. The amount of acetic acid to be used may be in the range of 10 to 150 mol per mol of the compound (13), and preferably 20 to 100 mol per mol of the compound. The amount of acetic anhydride to be used may be in the range of 10 to 150 mol per mol of the compound (13), and preferably 20 to 100 mol per mol of the compound. The reaction temperature is preferably in the range of 0°C to 100°C. The reaction time varies depending on the kind of raw materials and the reaction temperature, and is preferably between 1 and 48 hours.

### Step 2:

Process for producing a compound represented by the following general formula (15) by halogenating compound (14). 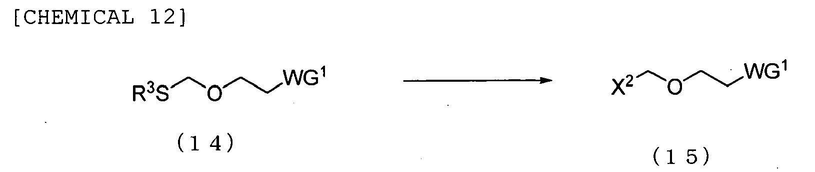 In the general formula (14) and (15), WG¹ and R³ have the same meanings as above. X² represents halogen.
Compound (15) is an ether compound (11) wherein L is halogen.
Examples of the "halogen" related to the X² may include the same ones as those illustrated for the "halogen" related to the above-mentioned modified form of B_{Z}.
The step can be carried out by well-known methods (e.g., T. Benneche et al., Synthesis, 762 (1983)). The solvent to be used is not specifically limited unless it is involved in the reaction, and may include, for example, halogenated hydrocarbon such as methylene chloride, chloroform, carbon tetrachloride, 1,2-dichloroethane.
Examples of the "halogenating agent" may include sulfuryl chloride and phosphorus oxychloride. The amount of the halogenating agent to be used may suitably be in the range of 0.8 to 20 mol per mol of the compound (14), and preferably 1 to 10 mol per mol of the compound. The reaction temperature is preferably in the range of 0°C to 100°C. The reaction time varies depending on the kind of raw materials and the reaction temperature, and is preferably between 30 minutes and 24 hours.

### Step 3:

Process for producing a compound represented by the following general formula (16), by arylthiolating the compound (15). 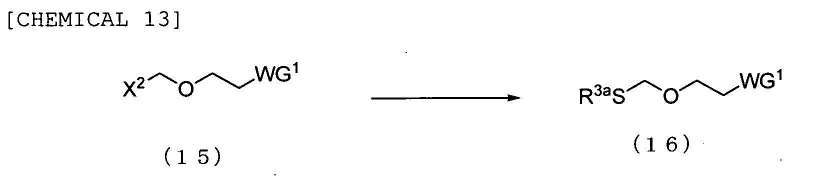 In the general formula (15) and (16), WG¹ and X² have the same meanings as above. R^{3a} represents aryl.
Compound (16) is an ether compound (11), wherein L is an arylthio group.
Examples of "aryl" related to R^{3a} may include the same ones as those illustrated for the "aryl" related to the above-mentioned modified form of B_{Z}.
The step can be carried out by a known method. The solvent to be used is not specifically limited unless it is involved in the reaction, and may include, for example, methylene chloride and acetonitrile. Examples of the "arylthiolating reagent" may include thiophenol and 4-methylbenzenethiol. The amount of the arylthiolating reagent to be used may be in the range of 0.8 to 20 mol per mol of the compound (15), and preferably 1 to 5 mol per mol the compound. The reaction temperature is preferably in the range of 0°C to 100°C. The reaction time varies depending on the kind of raw materials and the reaction temperature, and is preferably between 1 and 48 hours.

### Step 4:

Process for producing a compound represented by the following general formula (17) by oxidizing the compound (14). 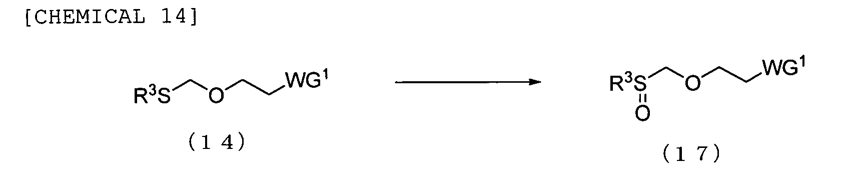 In the general formula (16) and (19), WG¹ and R³ have the same meanings as above.
The compound (19) is a compound (14), wherein L is an alkylsulfoxide group. Examples of the "alkyl" related to R³ may include the same ones as those illustrated for the "alkyl" related to the above-mentioned modified form of B_{Z}.
The step can be carried out by a known method. The solvent to be used is not specifically limited unless it is involved in the reaction, and may include, for example, methylene chloride, chloroform and methanol. Examples of the "oxidizing agent" may include m-chloroperbenzoic acid, metaperiodate salt and hydrogen peroxide. The amount of the oxidizing agent to be used may be in the range of 0.8 to 10 mol per mol of the compound (14), and preferably 1 to 2 mol per mol of the compound. The reaction temperature is preferably in the range of 0°C to 100°C. The reaction time varies depending on the kind of raw materials and the reaction temperature, and is preferably between 1 and 48 hours.

When compound (15) is used as the alkylating agent, the step can be performed as follows.
The step can be performed by allowing the alkylating agent and a base with ribonucleic acid derivative (6), which is commercially available or is synthesized according to a known method. The solvent to be used is not specifically limited unless it is involved in the reaction, and may include, for example, a halogenated hydrocarbon such as methylene chloride, chloroform, carbon tetrachloride and 1,2-dichloroethane. The amount of the alkylating agent to be used may be in the range of 0.8 to 20 mol per mol of the ribonucleic acid derivative (6), and preferably 1 to 10 mol per mol of the compound.
In the step, the alkylating agent may be reacted through the intermediate produced by reacting a metal reagent and a base with ribonucleic acid derivative (6), if necessary. Examples of the "metal reagent" may include dibutylstannyl dichloride. The amount of the metal reagent to be used may be in the range of 0.8 to 20 mol per mol of the ribonucleic acid derivative (6), and preferably 1 to 10 mol per mol of the compound. Examples of the "base" may include organic bases such as pyridine, 2,6-dimethylpyridine, 2,4,6-trimethylpyridine, N-methylimidazole, triethylamine, tributylamine, N,N-diisopropylethylamine and 1,8-diazabicyclo [5.4.0]-7-undecene. The amount of the base to be used may be in the range of 0.8 to 20 mol per mol of the ribonucleic acid derivative (6), and preferably 1 to 10 mol per mol of the compound. The reaction temperature is preferably in the range of 0°C to 120°C. The reaction time varies depending on the kind of raw materials and the reaction temperature, and is preferably between 30 minutes and 24 hours.

When compound (14) or (16) is used as the alkylating reagent, the step can be performed as follows.
The step can be performed according to a known method (e. g., M. Matteucci, Tetrahedron Letters, Vol. 31, 2385 (1990)) by reacting the alkylating reagent, an acid and a reagent for halogenating the sulfur atom on a ribonucleic acid derivative (6), which is commercially available or is synthesized by a known method. The amount of the alkylating reagent to be used may be in the range of 0.8 to 5 mol per mol of the ribonucleic acid derivative (6), and preferably 1 to 3 mol per mol of the compound.
Examples of the "acid" may include trifluoromethanesulfonic acid, silver trifluoromethanesulfonate and trimethylsilyl trifluoromethanesulfonate. The amount of the acid to be used may be in the range of 0.01 to 20 mol per mol of the ribonucleic acid derivative (6), and preferably 0.02 to 10 mol per mol of the compound. The solvent to be used is not specifically limited unless it is involved in the reaction, and may include, for example, methylene chloride, chloroform, carbon tetrachloride, 1,2-dichloroethane, benzene, toluene, xylene, THF, acetonitrile and mixtures thereof. Examples of the "reagent for halogenating a sulfur atom" to be used in the step may include N-bromosuccinimide (NBS) and N-iodosuccinimide (NIS). The amount of the reagent for halogenating a sulfur atom to be used may be in the range of 0.8 to 10 mol per mol of the ribonucleic acid derivative (6), and preferably 1 to 5 mol per mol of the compound. The reaction temperature is preferably in the range of -78°C to 30°C. The reaction time varies depending on the kind of raw materials and the reaction temperature, and is preferably between 5 minutes and 5 hours.

When the compound (17) is used as the alkylating reagent, the step can be performed as follows.
The step can be performed by allowing the alkylating reagent, an acid anhydride and a base with the ribonucleic acid derivative (6), which is commercially available or is synthesized according to a known method. The amount of the alkylating reagent to be used may be in the range of 0.8 to 5 mol per mol of the ribonucleic acid derivative (6), and preferably 1 to 3 mol per mol of the compound. Examples of the "acid anhydride" may include trifluoromethanesulfonic anhydride and acetic anhydride. The amount of the acid anhydride to be used may be in the range of 0.01 to 20 mol per mol of the ribonucleic acid derivative (6), and preferably 0.02 to 10 mol per mol of the compound. Examples of the "base" may include tetramethylurea and collidine. The amount of the base to be used may be in the range of 0.01 to 20 mol per mol of the ribonucleic acid derivative (6), and preferably 0.02 to 10 mol per mol of the compound. The solvent to be used is not specifically limited unless it is involved in the reaction, and may include, for example, methylene chloride, chloroform, carbon tetrachloride, 1,2-dichloroethane and mixtures thereof. The reaction temperature is preferably in the range of -78°C to 30°C. The reaction time varies depending on the kind of raw materials and the reaction temperature, and is preferably between 5 minutes and 24 hours.

### (2) Step b:

Process for producing a ribonucleic acid derivative represented by the following general formula (7) by allowing dimethylsulfoxide, acetic acid and acetic anhydride to act on the ribonucleic acid derivative (6), being independent of Step a. 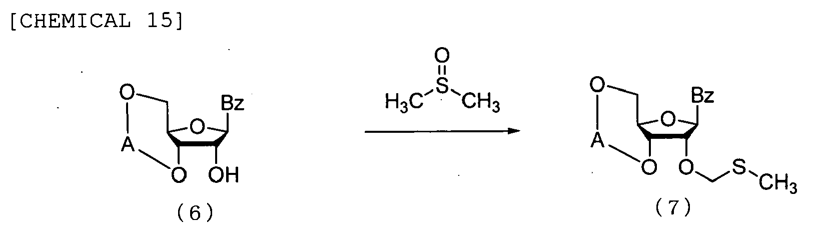 In the general formula (6) and (7), A and B_{Z} have the same meanings as above.
The step can be performed by reacting dimethylsulfoxide, acetic acid and acetic anhydride with a ribonucleic acid derivative (6), which is commercially available or is synthesized according to a known method. The amount of the dimethylsulfoxide to be used may be in the range of 10 to 200 mol per mol of the ribonucleic acid derivative (6), and preferably 20 to 100 mol per mol of the compound. The amount of the acetic acid to be used may be in the range of 10 to 150 mol per mol of the ribonucleic acid derivative (6), and preferably 20 to 100 mol per mol of the compound. The amount of the acetic anhydride to be used may be in the range of 10 to 150 mol per mol of the ribonucleic acid derivative (6), and preferably 20 to 100 mol per mol of the compound. The reaction temperature is preferably in the range of 10°C to 50°C. The reaction time varies depending on the kind of raw materials and the reaction temperature, and is preferably between 30 minutes and 24 hours.

### (3) Step c:

Process for producing a ribonucleic acid derivative represented by the following general formula (1), wherein an ether-type protecting group which can be removed under neutral conditions is introduced at the 2'-hydroxyl position, by allowing an alcohol compound represented by the following general formula (8), an acid and a reagent for halogenating a sulfur atom to act on a ribonucleic acid derivative (7) produced by Step b. 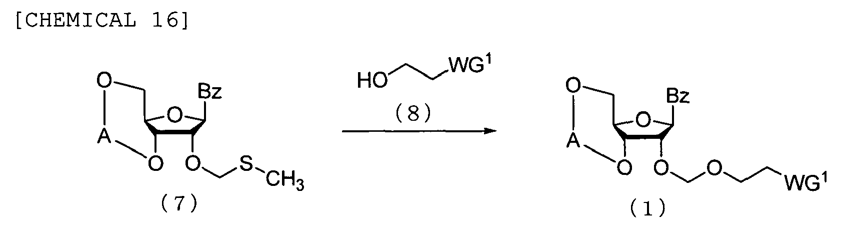 In the general formula (7), (8) and (1), A, B_{Z} and WG¹ have the same meanings as above.
The step can be performed by allowing the alcohol compound (8), an acid and a reagent for halogenating the sulfur atom on the ribonucleic acid derivative (7) according to a known method. The solvent to be used is not specifically limited unless it is involved in the reaction, and may include, for example, methylene chloride, chloroform, carbon tetrachloride, 1,2-dichloroethane, benzene, toluene, xylene, THF, acetonitrile and mixtures thereof. The amount of the alcohol compound (8) to be used may be in the range of 0.8 to 20 mol per mol of the ribonucleic acid derivative (7), and preferably 1 to 10 mol per mol of the compound. Examples of the "acid" may include trifluoromethanesulfonic acid, silver trifluoromethanesulfonate and trimethylsilyl trifluoromethanesulfonate. Examples of the "reagent for halogenating a sulfur atom" may include N-bromosuccinimide (NBS) and N-iodosuccinimide (NIS). The amount of the reagent for halogenating a sulfur atom to be used may be in the range of 0.1 to 20 mol per mol of the ribonucleic acid derivative (7), and preferably 0.2 to 10 mol per mol of the compound. The reaction temperature is preferably in the range of -100°C to 20°C. The reaction time varies depending on the kind of raw materials and the reaction temperature, and is preferably between 5 minutes and 12 hours.

### (4) Step d:

Process for producing a ribonucleic acid derivative represented by the following general formula (3), characterized by removing silyl protecting groups for the 3'-hydroxyl group and the 5'-hydroxyl group of a ribose of a ribonucleic acid derivative represented by the following general formula (1), allowing a salt of a tertiary amine represented by the following general formula (2) with hydrofluoric acid or a mixture of the tertiary amine and hydrofluoric acid to act on the ribonucleic acid derivative. 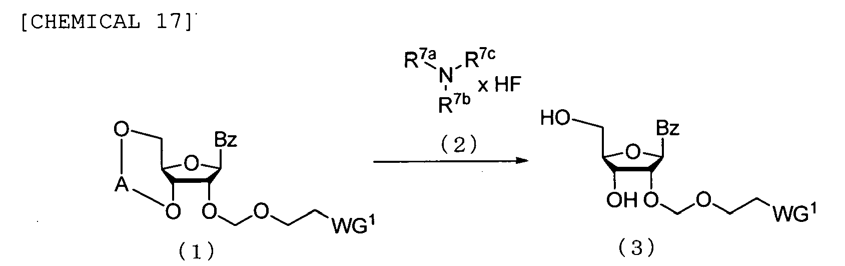 In the general formula (1), (2) and (3), A, B_{Z}, R^{7a}, R^{7b}, R^{7c}, WG¹ and x have the same meanings as above.
This step can be performed by allowing a salt of a tertiary amine represented by the above general formula (2) with hydrofluoric acid or a mixture of the tertiary amine and hydrofluoric acid to react on the ribonucleic acid derivative (1) which is dissolved in a suitable solvent. Further, in some cases, this step can be performed by using a mixed reagent including a suitable acid to a salt of a tertiary amine with hydrofluoric acid or a mixture of the tertiary amine and hydrofluoric acid. Examples of the "acid" to be used may include acetic acid, hydrochloric acid, and hydrosulfuric acid. The amount of the acid to be used may be in the range of 0.8 to 10 mol per mol of the ribonucleic acid derivative (1), and preferably 1 to 1.5 mol per mol of the compound. The solvent to be used may include, for example, THF, acetonitrile, methanol, isopropanol and toluene. In particular, THF or acetonitrile is preferred. The amount of the "salt of a tertiary amine with hydrofluoric acid or a mixture of the tertiary amine and hydrofluoric acid" to be used in this step varies depending on the kind of the ribonucleic acid derivative (1), a salt of a tertiary amine with hydrofluoric acid, a mixture of the tertiary amine and hydrofluoric acid, and the reaction solvent, and is suitably in the range of 1 to 10 mol per mol of the ribonucleic acid derivative (1), and preferably 1.2 to 1.5 mol per mol of the compound. The reaction temperature is preferably in the range of 0°C to 80°C. The reaction time varies depending on the kind of the ribonucleic acid derivative (1), a salt of a tertiary amine with hydrofluoric acid, a mixture of the tertiary amine and hydrofluoric acid, and the reaction solvent, and is suitably between 30 minutes and 10 hours. After the reaction is terminated, the ribonucleic acid derivative (3) can be obtained as a precipitate, by cooling the reaction mixture as it is or with the addition of water if necessary. The amount of the additional water is suitably in the range of 0.06 to 1 mol per mol of the solvent to be used, preferably 0.06 to 1 mol per mol of the solvent, and more preferably 0.07 to 1 mol per mol of the solvent.

### (5) Step e:

Process for producing a ribonucleic acid derivative (10) by introducing a protecting group (R¹), which can be removed under acidic conditions, into the 5'-hydroxyl group of the ribonucleic acid derivative (3) produced by Step d. 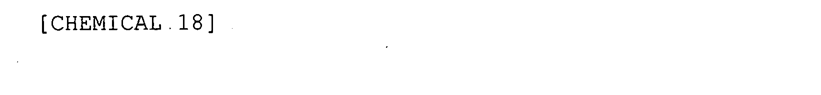 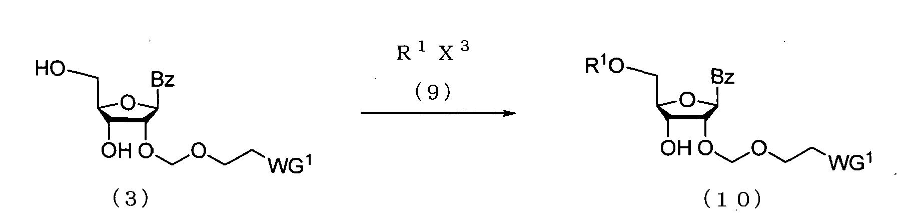 In the general formula (3), (9) and (10), B_{Z}, R¹ and WG¹ have the same meanings as above. X³ represents halogen.
Examples of the "halogen" related to the X³ may include the same ones as those illustrated for the "halogen" related to the above-mentioned modified form of B_{Z}.
The step can be performed by allowing R¹X³ (9) with a ribonucleic acid derivative (3) according to a known method. The amount of R¹X³ (9) to be used may be in the range of 0.8 to 20 mol per mol of the ribonucleic acid derivative (3), and preferably 1 to 10 mol per mol of the compound. The solvent to be used is not specifically limited unless it is involved in the reaction, and may include, for example, acetonitrile and THF. Examples of the "base" may include organic bases such as pyridine, 2,6-dimethylpyridine, 2,4,6-trimethylpyridine, N-methylimidazole, triethylamine, tributylamine, N,N-diisopropylethylamine and 1,8-diazabicyclo[5.4.0]-7-undecene.
The amount of the base to be used may be in the range of 0.8 to 20 mol per mol of the ribonucleic acid derivative (3), and preferably 1 to 10 mol per mol of the compound. The reaction temperature is preferably in the range of 0°C to 120°C. The reaction time varies depending on the kind of raw materials and the reaction temperature, and is preferably between 30 minutes and 24 hours.

### (6) Step f:

Process for producing the phosphoramidite compound (A), wherein 3'-hydroxyl group is phosphoramidited, by allowing a phosphoramiditing reagent and an activating agent, if necessary, to act on the ribonucleic acid derivative (10) produced by Step e. 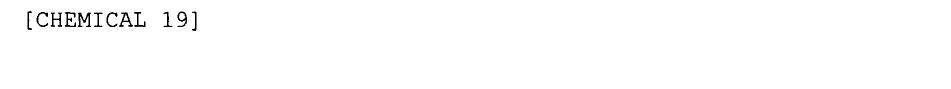 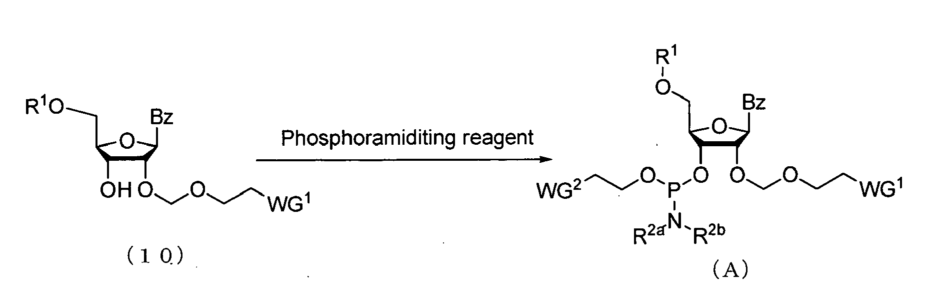 In the general formula (10) and (A), B_{Z}, R¹, R^{2a}, R^{2b}, WG¹ and WG² have the same meanings as above.
Examples of the "phosphoramiditing reagent" may include a compound represented by the following general formula (12a) and (12b). 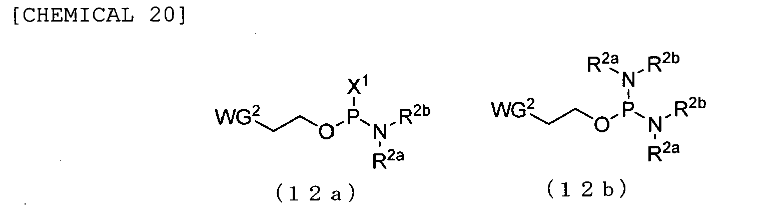 In the general formula (12a) and (12b), R^{2a}, R^{2b} and WG² have the same meanings as above. X¹ represents halogen.
Examples of the "halogen" related to the X¹ may include the same ones as those illustrated for the "halogen" related to the above-mentioned modified form of B_{Z}.
The step is a reaction for phosphoramiditing the 3'-hydroxyl group by reacting the phosphoramiditing reagent with a ribonucleic acid derivative (10), and can be performed according to a known method. An activating agent can be used if necessary. The solvent to be used is not specifically limited unless it is involved in the reaction, and may include, for example, acetonitrile and THF. The amount of the phosphoramiditing reagent to be used may be in the range of 0.8 to 20 mol per mol of the ribonucleic acid derivative (10), and preferably 1 to 10 mol per mol of the compound. Examples of the "activating agent" may include 1H-tetrazole, 5-ethylthiotetrazole, 5-benzylmercapto-1H-tetrazole, 4,5-dichloroimidazole, 4,5-dicyanoimidazole, benzotriazole triflate, imidazole triflate, pyridinium triflate, N,N-diisopropylethylamine and 2,4,6-collidine/N-methylimidazole. The amount of the activating agent to be used may be in the range of 0.8 to 20 mol per mol of the ribonucleic acid derivative (10), and preferably 1 to 10 mol per mol of the compound. The reaction temperature is preferably in the range of 0°C to 120°C. The reaction time varies depending on the kind of raw materials and the reaction temperature, and is preferably between 30 minutes and 24 hours.
The phosphoramidite compound (A) thus produced can be isolated and purified by a method known per se, such as concentration, liquid phase conversion, partition, solvent extraction, crystallization, recrystallization, fractional distillation or chromatography.

### II. A method for producing the oligoribonucleic acid (A)

Oligoribonucleic acid represented by the following general formula (B) (hereinafter referred to as "oligoribonucleic acid (B)") can be produced by using the phosphoramidite compound (A).
The details are described below. 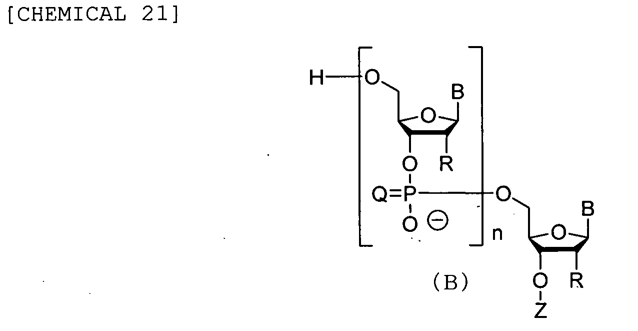 In the general formula (B), each B represents independently nucleobase or a modified form thereof. Each Q independently represents O or S. Each R independently represents H, hydroxyl, halogen, alkoxy, alkylthio, alkylamino, dialkylamino, alkenyloxy, alkenylthio, alkenylamino, dialkenylamino, alkynyloxy, alkynylthio, alkynylamino, dialkynylamino or alkoxyalkyloxy, and at least one R is hydroxyl. Z represents H, a phosphate group or a thiophosphate group. n represents an integer in the range of 1 to 200. n is preferably an integer in the range of 10 to 100, and more preferably an integer in the range of 15 to 50.
Examples of the "nucleobase" related to B is not particularly limited as long as it is a nucleobase to be used in the synthesis of a nucleic acid, and examples thereof may include pyrimidine bases such as cytosine, uracil and thymine, purine bases such as adenine and guanine. The "modified form" of B is a group in which a nucleobase has been substituted by an arbitrary substituent.
Examples of the "substituent" related to the modified form of B may include halogen, acyl, alkyl, arylalkyl, alkoxy, alkoxyalkyl, hydroxyl, amino, monoalkylamino, dialkylamino, carboxy, cyano and nitro. The modified form of B may be substituted by 1 to 3 of these substituents at arbitrary positions.
Examples of the "halogen", "acyl", "alkyl", "arylalkyl", "alkoxy", "alkoxyalkyl" "amino", "monoalkylamino" or "dialkylamino" related to the modified form of B may include the same ones as those related to the above-mentioned modified form of B_{Z}, respectively. Examples of the "halogen", "alkoxy", "alkylamino" and "dialkylamino" related to R may include the same ones as those related to the above-mentioned modified form of B_{Z} mentioned above, respectively.
Examples of the "alkyl" moiety of the "alkoxyalkyloxy" and "alkylthio" related to R may include the same ones as those illustrated for the "alkyl" related to the above-mentioned modified form of B_{Z}.
Examples of the "alkoxy" moiety of the "alkoxyalkyloxy" related to R may include the same ones as those illustrated for the "alkoxy" related to the above-mentioned modified form of B_{Z}.
Examples of the "alkenyl" of the "alkenyloxy", "alkenylthio", "alkenylamino" and "dialkenylamino" related to R may include straight or branched alkenyl having 2 to 6 carbon atoms. Specifically, the alkenyl may include, for example, vinyl, allyl, 1-propenyl, isopropenyl, 1-butenyl, 2-butenyl, 1-pentenyl and 1-hexenyl. Examples of the "alkynyl" moiety of the "alkynyloxy", "alkynylthio", "alkynylamino" and "dialkynylamino" related to R may include straight or branched alkynyl having 2 to 4 carbon atoms. Specifically, the alkynyl may include, for example, ethynyl, 2-propynyl and 1-butynyl.
A method for producing oligo-RNA (B) using phosphoramidite compound (A) can be performed by a known method, for example, by condensing a nucleic acid monomer compound to the direction from 3' to 5' step by step according to the following Steps A to H.
In the method for producing the oligo-RNA mentioned above, it is possible to produce an oligo-RNA (B) wherein one or more Rs are hydroxyl group. For example, in process B mentioned below, it is possible to produce an oligo-RNA (B) in which all Rs of are hydroxyl groups, by using solely the phosphoramidite compound (A) as a nucleic acid monomer compound. Compounds and reagents to be used in the following steps except the phosphoramidite compound (A) are not particularly limited as long as they are generally used in syntheses of oligo-RNAs or oligo-DNAs. In addition, all the steps can be performed by using an automatic synthesizer for DNA or in manual as in the case of using conventional reagents for synthesizing a nucleic acid. The use of an automatic synthesizer is desirable from the point of view of the simplicity and ease of the method and the accuracy of the synthesis. Compounds and reagents described in the following Steps A to G except a nucleic acid monomer compound are not particularly limited as long as they are generally used in syntheses of oligo-DNAs or oligo-RNAs.

### (1) Step A:

Process for producing a (oligo)nucleic acid derivative represented by the following general formula (19) by removing the 5'-hydroxyl group from a (oligo)nucleic acid derivative represented by the following general formula (18) by allowing an acid. 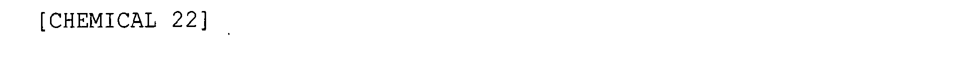 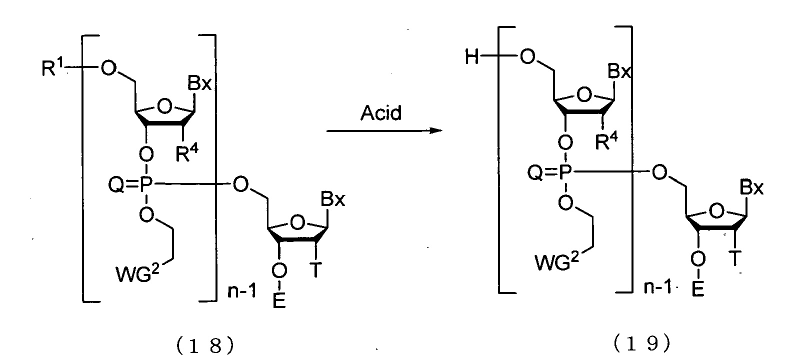 In the general formulae (18) and (19), each Q independently has the same meanings as above. n, R¹ and WG² have the same meanings as above.
Each B_{X} independently represents a nucleobase which may have protecting groups, or a modified form thereof.
Each R⁴ independently represents H, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or the substituent represented by the following general formula (20). 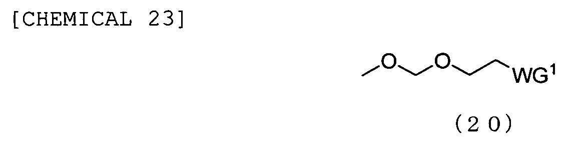 In the general formula (20), WG¹ has the same meanings as above.
E represents acyl and a substituent represented by the following general formula (21). 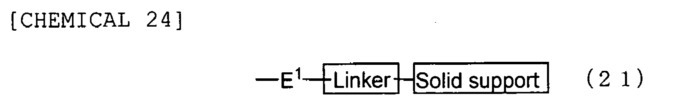 In the general formula (21), E¹ represents a single bond or a substituent represented by the following general formula (22). 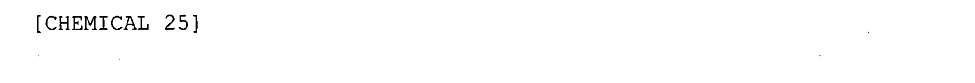 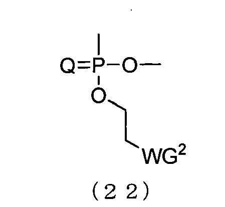 In the general formula (22), Q and WG² have the same meanings as above.
T represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy, an substituent represented by the above general formula (20) or a substituent represented by the above general formula (21), with the proviso that either E or T is a substituent (21).
Examples of the "nucleobase" related to B_{X} is not particularly limited as long as it is a nucleobase to be used in the synthesis of a nucleic acid, and examples thereof may include pyrimidine bases such as cytosine, uracil, and thymine, purine bases such as adenine and guanine.
The "nucleobase" related to B_{X} may be protected, and particularly in the case of a nucleobase having an amino group such as adenine, guanine or cytosine, the amino group thereof is preferably protected. The "protecting group of amino group" is not particularly limited as long as it is a protecting group to be used as a protecting group of a nucleic acid, and examples thereof may include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene.
The "modified form" related to B_{X} is a group in which a nucleobase has been substituted by an arbitrary substituent. Examples of the "substituent" related to the modified form of B_{X} may include halogen, acyl, alkyl, arylalkyl, alkoxy, alkoxyalkyl, hydroxyl, amino, monoalkylamino, dialkylamino, carboxy, cyano and nitro. The modified form of B may be substituted by 1 to 3 of these substituents at arbitrary positions.
Examples of the "halogen", "acyl", "alkyl", "arylalkyl", "alkoxy", "alkoxyalkyl", "monoalkylamino" or "dialkylamino" related to the modified form of B_{X} may include the same ones as those related to the above-mentioned modified form of B_{Z}.
Examples of the "halogen", "alkoxy", "alkylamino" or "dialkylamino" related to R⁴ may include the same ones as those related to the above-mentioned modified form of B_{Z}.
Examples of the "alkyl" moiety of the "alkoxyalkyloxy" and "alkylthio" related to R⁴ may include the same ones as those illustrated for the "alkyl" related to the above-mentioned modified form of B_{Z}.
Examples of the "alkoxy" moiety of the "alkoxyalkyloxy" related to R⁴ may include the same ones as those illustrated for the "alkoxy" related to the above-mentioned modified form of B_{Z}.
Examples of the "alkenyl" moiety of "alkenyloxy", "alkenylthio", "alkenylamino", "dialkenylamino" related to R⁴ may include the same ones as those illustrated for the "alkenyl" related to the above-mentioned R.
Examples of the "alkynyl" moiety of the "alkynyloxy" "alkynylthio", "alkynylamino" and "dialkynylamino" related to R⁴ may include the same ones as those illustrated for the "alkynyl" related to the above-mentioned R. The "alkylamino", "alkenylamino" or "alkynylamino" related to R⁴ may be protected. The protecting group of the amino group is not particularly limited as long as it is a protecting group to be used as a protecting group of an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particularly, trifluoroacetyl is preferred.
Examples of the "acyl" related to the E may include the same ones as those illustrated for the "acyl" related to the above-mentioned modified form of B_{Z}.
Examples of the "acyl" moiety of "acyloxy" related to the T may include the same ones as those illustrated for the "acyl" related to the above-mentioned modified form of B_{Z}.
Examples of the "halogen", "alkoxy", "alkylamino" or "dialkylamino" related to the T may include the same ones as those related to the above-mentioned modified form of B_{Z}.
Examples of the "alkyl" moiety of the "alkoxyalkyloxy" and "alkylthio" related to the T may include the same ones as those illustrated for the "alkyl" related to the above-mentioned modified form of B_{Z}.
Examples of the "alkoxy" moiety of the "alkoxyalkyloxy" related to the T may include the same ones as those illustrated for the "alkoxy" related to the above-mentioned modified form of B_{Z}.
Examples of the "alkenyl" moiety of "alkenyloxy", "alkenylthio", "alkenylamino" and "dialkenylamino" related to the T may include the same ones as those illustrated for the "alkenyl" related to the above-mentioned R.
Examples of the "alkynyl" moiety of "alkynyloxy" "alkynylthio", "alkynylamino", "alkylamino" and "dialkynylamino" related to the T may include the same ones as those illustrated for the "alkynyl" related to the above-mentioned A.
The "alkylamino", "alkenylamino" and "alkynylamino" of T may be protected. The protecting group of the amino group is not particularly limited as long as it is a protecting group to be used as a protecting group of an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. In particular, trifluoroacetyl is preferred. The step is performed by allowing an acid with a compound represented by the following formula (23a), (23b) (a nucleic acid derivative (18) wherein n is 1) which is attached to the solid support, or an oligo-RNA or an oligo-DNA produced by performing the operations of Step A to Step D (oligonucleic acid derivative (18) wherein n is 2 to 100) which is attached to the solid support (hereinafter referred to as the "oligonucleic acid attached the solid support"). 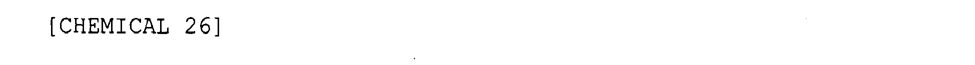 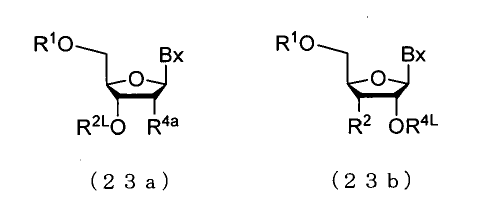 In the general formulae (23a) and (23b), B_{X} and R¹ have the same meanings as above. R^{2L} and R^{4L} represent substituent (21). R² represents acyloxy. R^{4a} represents H, acyloxy, halogen, alkoxy, alkylthio, alkylamino, dialkylamino, alkenyloxy, alkenylthio, alkenylamino, dialkenylamino, alkynyloxy, alkynylthio, alkynylamino, dialkynylamino, alkoxyalkyloxy or substituent (20).
Examples of the "acyl" moiety of the "acyloxy" relate to R² and R^{4a} may include the same ones as those illustrated for the "acyl" related to the above-mentioned modified form of B_{Z}.
Examples of the "halogen", "alkoxy", "alkylamino" or "dialkylamino" related to R^{4a} may include the same ones as those related to the above-mentioned modified form of B_{Z}.
Examples of the "alkyl" moiety of "alkoxy alkyloxy" and "alkylthio" related to R^{4a} may include the same ones as those illustrated for the "alkyl" related to the above-mentioned modified form of B_{Z}.
Examples of the "alkoxy" moiety of the "alkoxyalkyloxy" related to R^{4a} may include the same ones as those illustrated for the "alkoxy" related to the above-mentioned modified form of B_{Z}.
Examples of the "alkenyl" moiety of "alkenyloxy", "alkenylthio", "alkenylamino" and "dialkenylamino" related to R^{4a} may include the same ones as those illustrated for the alkenyl related to the above-mentioned R.
Examples of the "alkynyl" moiety of the "alkynyloxy", "alkynylthio", "alkynylamino" and "dialkynylamino" related to R^{4a} may include the same ones as those illustrated for the "alkynyl" related to the above-mentioned R.
The "amino", "alkylamino", "alkenylamino" and "alkynylamino" of R^{4a} may be protected. The protecting group of the amino group is not particularly limited as long as it is a protecting group to be used as a protecting group of an amino group, and examples thereof may include trifluoroacetyl, benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene. Particularly, trifluoroacetyl is preferred.
Examples of the "solid support" may include a controlled-pore glass (CPG), an oxalyl-controlled pore glass (see, for example, Alul et al., Nucleic Acids Research, Vol. 19, 1527 (1991)), TentaGel support-amino polyethylene glycol derivatization support (see, for example, Wright et al., Tetrahedron Letters, Vol.34, 3373 (1993)) and a copolymer of porous polystyrene and divinylbenzene.
Examples of the "linker" may include 3-aminopropyl, succinyl, 2,2'-diethanol sulfonyl and a long chain alkylamino (LCAA).
The nucleic acid derivative (23a), nucleic acid derivative (23b) are attached to the solid support, which are produced according to a known method or are commercially available, and examples of a preferable embodiment are a nucleic acid derivative represented by the following general formula (24), (25). 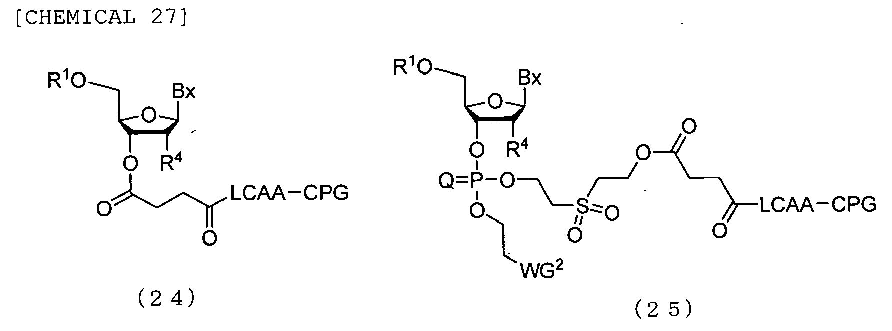 In the general formulae (24) and (25), B_{X}, Q, R¹, R⁴ and WG² have the same meanings as above.
The nucleic acid derivative (24) and (25) wherein R⁴ is a substituent (20) can be produced from a phosphoramidite compound (A) according to a known method.
Examples of the "acid" to be used in the step may include trifluoroacetic acid, dichloroacetic acid and trichloroacetic acid.
The acid to be used in the step can be diluted in a suitable solvent so as to be of a concentration of 1 to 5 %. The solvent is not specifically limited unless it is involved in the reaction, and may include methylene chloride, acetonitrile, water and an arbitrary mixture thereof. The reaction temperature in the reaction is preferably in the range of 20°C to 50°C. The reaction time varies depending on the kind of the oligonucleic acid derivative (18), the acid and the reaction temperature, and is preferably between 1 minute and 1 hour. The amount of the reagent to be used is preferably in the range of 0.8 to 100 mol per mol of the (oligo)nucleic acid derivative attached to the solid phase support, and more preferably 1 to 10 mol per mol of the compound attached to the solid support.

### (2) Step B:

Process for producing an oligonucleic acid derivative represented by the following general formula (26) by condensing a nucleic acid monomer compound with the oligonucleic acid derivative (19) produced by Step A using an activating agent. 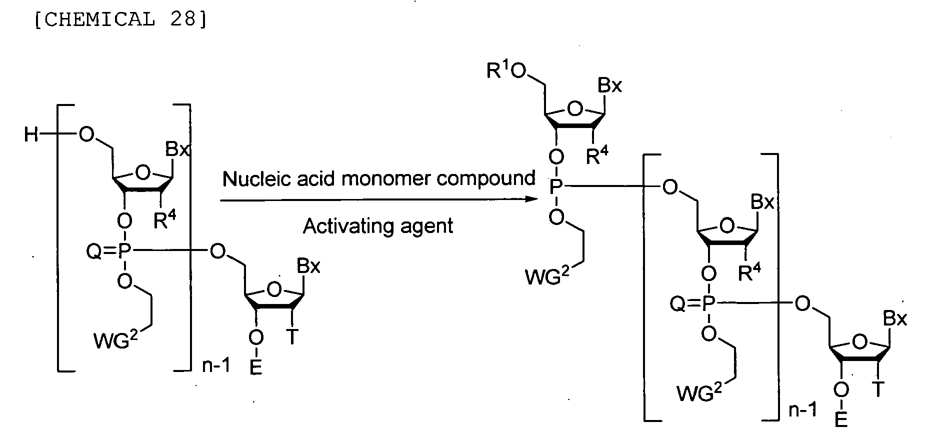 In the general formulae (19) and (26), each B_{X}, each Q, each R⁴ and each WG² independently have the same meanings as above. E, n, R¹ and T have the same meanings as above.
The step can be performed by allowing a nucleic acid monomer compound and an activating agent with an oligonucleic acid derivative attached to the solid phase support.
Examples of the "nucleic acid monomer compound", may include the phosphoramidite compound (A) and a nucleic acid derivative represented by the following general formula (27). 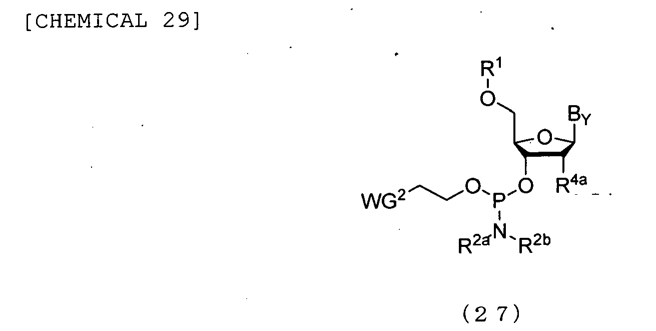 In the general formula (27), R¹, R^{2a}, R^{2b}, R^{4a} and WG² have the same meanings as above.
B_{Y} represents a nucleobase which may have protecting groups, or a modified form thereof. The "nucleobase" related to B_{Y} is not particularly limited as long as it is a nucleobase to be used in the synthesis of a nucleic acid, and examples thereof may include pyrimidine bases such as cytosine, uracil and thymine, and purine bases such as adenine and guanine. The "nucleobase" of B_{Y} may be protected, and particularly in the case of a nucleobase having an amino group, such as adenine, guanine or cytosine, the amino group thereof is preferably to be protected. The protecting group of amino group is not particularly limited as long as it is a protecting group to be used as a protecting group of a nucleic acid, and examples thereof may include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl and (dimethylamino)methylene.
The "modified form" related to B_{Y} is a group in which a nucleobase has been substituted by an arbitrary substituent. Examples of the "substituent" related to the modified form of B_{Y} may include halogen, acyl, alkyl, arylalkyl, alkoxy, alkoxyalkyl, hydroxyl, amino, monoalkylamino, dialkylamino, carboxy, cyano and nitro. The modified form of B may be substituted by 1 to 3 of these substituents at arbitrary positions.
Examples of the "halogen", "acyl", "alkyl", "arylalkyl", "alkoxy", "alkoxyalkyl", "monoalkylamino" or "dialkylamino" of the modified form of B_{Y} may include the same ones as those related to the above-mentioned modified form of B_{Z}.
Examples of the "activating agent" may include the same ones as those illustrated in the above description. The reaction solvent to be used is not specifically limited unless it is involved in the reaction, and may include, for example, acetonitrile and THF.
The reaction temperature is preferably in the range of 20°C to 50°C. The reaction time varies depending on the kind of an oligonucleic acid derivative (19), the kind of an activating agent to use and the reaction temperature, and preferably between 1 minute and 1 hour. The amount of the agent to be used is preferably in the range of 0.8-100 mol per mol of the oligonucleic acid derivative attached to the solid phase support, and more preferably 1 to 10 mol per mol of the compound attached to the solid support.

### (3) Step C:

Process for capping the 5'-hydroxyl group of the unreacted oligonucleic acid derivative (19) in Step B. 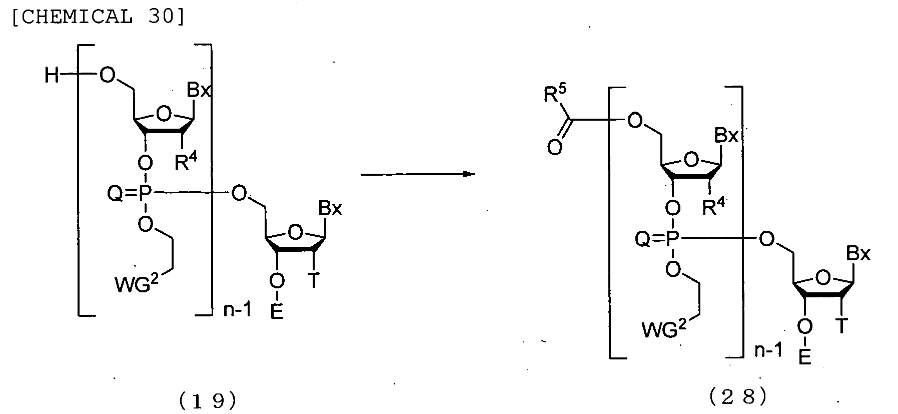 In the general formulae (19) and (28), each B_{X}, each Q, each R⁴, each WG² independently have the same meanings as above. E, n and T have the same meanings as above. R⁵ represents methyl, phenoxymethyl and tert-butylphenoxymethyl.
The step is a reaction for protecting the 5'-hydroxyl group unreacted in Step B, and can be performed by allowing a capping agent with an oligonucleic acid derivative attached to the solid phase support.
Examples of the "capping agent" may include acetic anhydride, phenoxyacetic anhydride and tert-butylphenoxyacetic anhydride. The capping agent to be used can be diluted in a suitable solvent so as to be of a concentration of 0.05 to 1 M. The reaction solvent to be used is not specifically limited unless it is involved in the reaction, and may include, for example, pyridine, methylene chloride, acetonitrile, THF and mixtures thereof. In addition, for example, 4-dimethylaminopyridine and N-methylimidazole can be used as a "reaction accelerator" in the step, if necessary. The reaction temperature in the reaction is preferably in the range of 20°C to 50°C. The reaction time varies depending on the kind of the oligonucleic acid derivative (19), the capping agent and the reaction temperature, and is preferably between 1 and 30 minutes. The amount of the capping agent to be used is preferably in the range of 0.8-100 mol per mol of the oligonucleic acid derivative attached to the solid phase support, and more preferably 1 to 10 mol per mol of the compound attached to the solid support.

### (4) Step D:

Process for converting a phosphite group into a phosphate group or thiophosphate group by reacting the oligonucleic acid derivative (26) produced in Step B with an oxidizing agent. 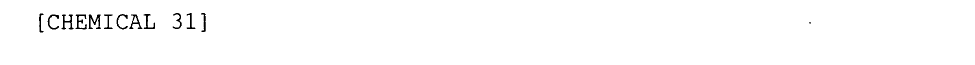 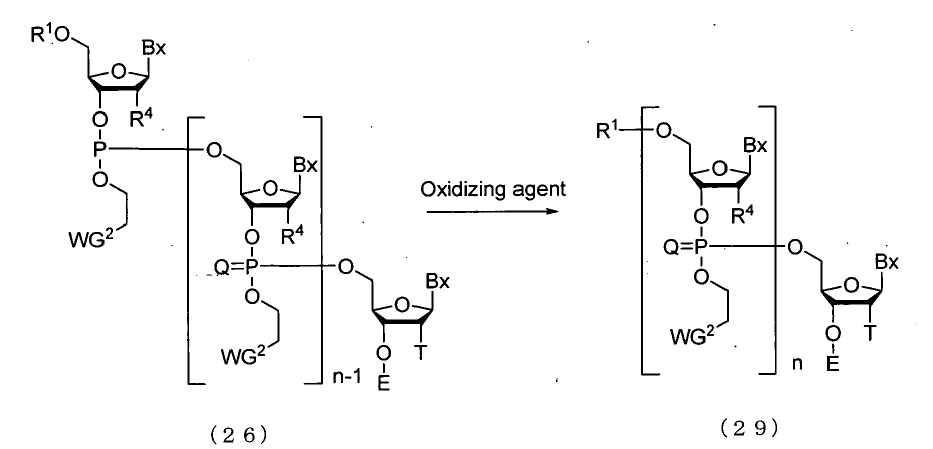 In the general formulae (26) and (29), each B_{X}, each Q, each R⁴ and each WG² independently have the same meanings as above. E, n, R¹ and T have the same meanings as above.
The step is a reaction for converting trivalent phosphorus to pentavalent phosphorus by using an oxidizing agent, and can be performed by allowing an oxidizing agent to react with an oligonucleic acid derivative attached to the solid phase support.
When phosphorus is oxidized with oxygen, examples of the "oxidizing agent" may include iodine and tert-butylhydroperoxide. In addition, the oxidizing agent to be used can be diluted in a suitable solvent so as to be of a concentration of 0.05 to 2 M. The reaction solvent to be used is not specifically limited unless it is involved in the reaction, and may include, for example, pyridine, tetrahydrofuran, water and mixtures thereof. For example, iodine / water / pyridine - THF, iodine / pyridine - acetic acid and a peroxidation agent (tert-butylhydroperoxide / methylene chloride and the like) can be used.
In addition, when phosphorus is oxidized with sulfur, examples of the "oxidizing agent" may include sulfur, Beaucage reagent (3H-1,2-benzodithiol-3-on-1,1-dioxide) and 3-amino-1,2,4-dithiazole-5-thione (ADTT). The oxidizing agent to be used can be diluted in a suitable solvent so as to be of a concentration of 0.01 to 2 M. The reaction solvent to be used is not specifically limited unless it is involved in the reaction, and may include, for example, methylene chloride, acetonitrile, pyridine and mixtures thereof. The reaction temperature is preferably in the range of 20°C to 50°C. The reaction time varies depending on the kind of the oligonucleic acid derivative (26), the oxidizing agent and the reaction temperature, and is preferably between 1 and 30 minutes. The amount of the oxidizing agent to be used is preferably in the range of 0.8-100 mol per mol of the oligonucleic acid derivative attached to the solid phase support, and more preferably 10 to 50 mol per mol of the compound attached to the solid support.

### (5) Step E:

Process for cleaving the oligonucleic acid derivative (29) produced by Step D from the solid support, and then removing the protecting groups of each nucleobase and each phosphate group. 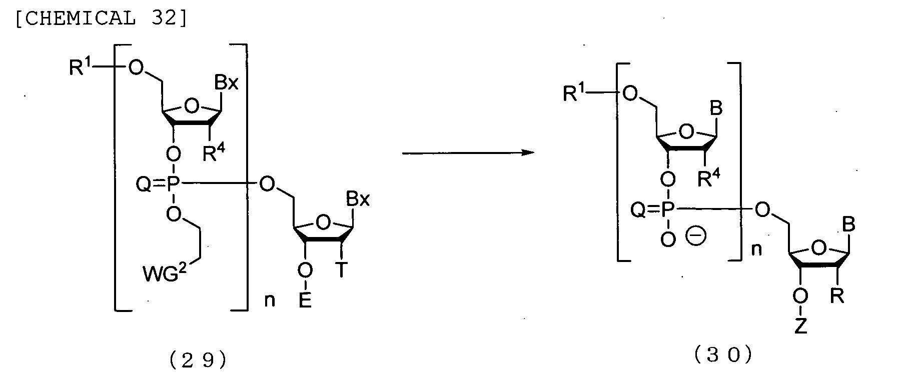 In the general formulae (29) and (30), each B, each B_{X}, each Q, each R⁴ and each WG² independently have the same meanings as above. E, R, R¹, n, T and Z have the same meanings as above.
The cleaving step is a reaction for cleaving an oligo-RNA having a desired chain length from solid phase support and linker with a cleaving agent, and is performed by adding a cleaving agent to the solid support which contains an oligo-RNA having a desired chain length. In the step, the protecting group of a nucleobase can be removed. Examples of the "cleaving agent" may include concentrated aqueous ammonia and methylamine. The cleaving agent to be used in the step may be diluted by, for example, water, methanol, ethanol, isopropyl alcohol, acetonitrile, THF and mixtures thereof. Among them, ethanol is preferred. The reaction temperature may be in the range of 15°C to 75°C, preferably it is 15°C to 30°C, and more preferably reaction temperature is 18°C to 25°C. The reaction time for deprotection varies depending on the kind of the oligonucleic acid derivative (9), the oxidizing agent and the reaction temperature, and may be in the range of 10 minutes to 30 hours, preferably 30 minutes to 24 hours, and more preferably 1 to 4 hours. The concentration of ammonium hydroxide in the solution to be used for deprotection may be 20 to 30 % by weight, preferably 25 to 30 % by weight, and more preferably 28 to 30 % by weight. The amount of the ammonium hydroxide to be used may be in the range of 1 to 100 mol per mol of the oligonucleic acid derivative attached to the solid phase support, and preferably 10 to 50 mol per mol of the compound attached to the solid support.

### (6) Step F:

Process for producing an oligonucleic acid derivative represented by the following general formula (31) by allowing a reagent for removing protecting group of 2'-hydroxyl group of each ribose to act on the oligonucleic acid derivative (30) produced in Step E. 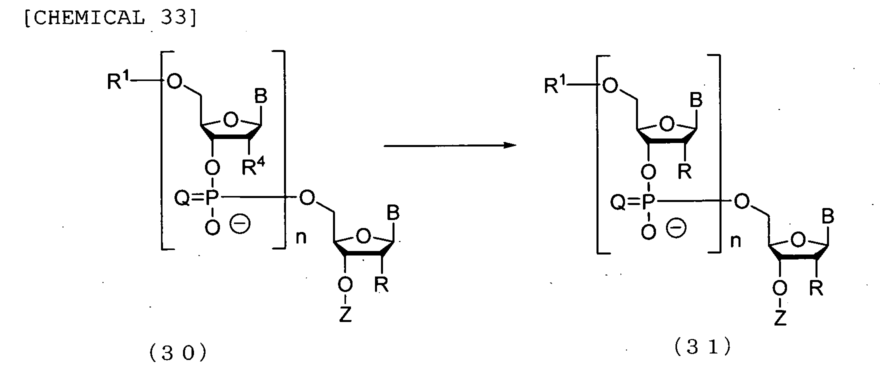 In the general formulae (30) and (31), each B, each Q, each R and each R⁴ independently have the same meanings as above. n, R¹ and Z are the same defined above.
The step can be performed by allowing the agent for removing the protecting group of the 2'-hydroxyl group to act on the oligonucleic acid derivative (30). The step for removing the protecting group of 2'-hydroxyl group is performed by using the reagent for removing the protecting group of the 2'-hydroxyl group such as tetrabutylammonium fluoride, and triethylamine / trihydrogenfluoride. The amount of the agent for removing the protecting group of the 2'-hydroxyl group may be in the range of 1 to 500 mol per mol of the protecting group to be removed, and preferably 5 to 10 mol per mol of the protecting group to be removed. The solvent to be used is not specifically limited unless it is involved in the reaction, and may include, for example, THF, N-methylpyrrolidone, pyridine, dimethylsulfoxide and mixtures thereof. The solvent to be used in the reaction may be in the range of 0.8 to 100 mol per mol of the agent for removing the protecting group of the 2'-hydroxyl group, and preferably 1 to 10 mol per mol of the agent for removing the protecting group of the 2'-hydroxyl group. The reaction temperature is preferably in the range of 20°C to 80°C. The reaction time varies depending on the kind of the oligonucleic acid derivative (30), the agent for removing the protecting group of the 2'-hydroxyl group to be used and the reaction temperature, and is preferably in the range of 1 hour to 100 hours. In addition, nitroalkane, alkylamine, amidine, thiol, thiol derivative and mixture thereof can be added as a scavenger of acrylonitrile, if necessary, to trap the acrylonitrile which is a by-product in the step.
Examples of the "nitroalkane" may include straight nitroalkane having 1 to 6 carbon atoms.
Specifically, the nitroalkane may include, for example, nitromethane.
Examples of the "alkylamine" may include straight alkylamine having 1 to 6 carbon atoms. Specifically, the "alkylamine" may include, for example, methylamine, ethylamine, n-propylamine, n-butylamine, n-pentylamine and n-hexylamine.
Examples of the "amidine" may include benzamidine and formamidine.
Examples of the "thiol" may include straight thiol having 1 to 6 carbon atoms.
Specifically, the "thiol" may include, for example, methanethiol, ethanethiol, 1-propanethiol, 1-butanthiol, 1-pentanethiol and 1-hexanthiol.
Examples of the "thiol derivative" may include alcohol and ether having the same or different straight alkylthiol having 1 to 6 carbon atoms. Specifically, the thiol derivative may include, for example, 2-mercaptoethanol, 4-mercapto-1-butanol, 6-mercapto-1-hexanol, mercaptomethyl ether, 2-mercaptoethyl ether, 3-mercaptopropyl ether, 4-mercaptobutyl ether, 5-mercaptopentyl ether and 6-mercaptohexyl ether.
The amount of the scavenger of acrylonitrile to be used varies depending on the kind of the oligonucleic acid derivative (30), and may be in the range of 0.8 to 500 mol per mol of 2-cyanoethoxymethyl substituting the 2'-hydroxyl group of each ribose of the oligonucleic acid derivative (30), and preferably 1 to 10 mol per mol.
It is possible to isolate and purify an oligo-RNA whose 5'-hydroxyl group is protected from the above reaction mixture with a known method, for example, extraction, concentration, neutralization, filtration, centrifugal separation, recrystallization, silica gel column chromatography, thin layer chromatography, reverse-phase ODS column chromatography, ion-exchange column chromatography, gel filtration column chromatography, dialysis, ultrafiltration and combinations thereof.

### (7) Step G:

Process for removing the 5'-hydroxyl group of the oligonucleic acid derivative (31) produced by Step F. 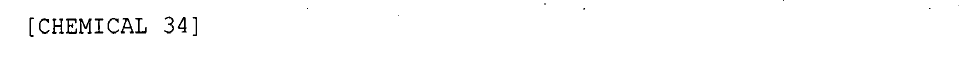 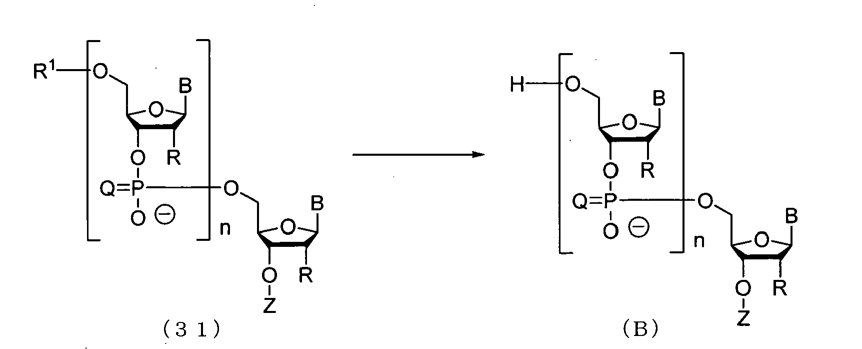 In the general formulae (31) and (B), each B, each Q and each R independently have the same meanings as above. n, R¹ and Z have the same meanings as above.
The step is a reaction for finally removing the protecting group of the 5'-hydroxyl group of the oligonucleic acid derivative (31), and can be performed by allowing an acid with the oligo-RNA having cleaved from the solid support.
Examples of the "acid" to be used in the step may include, trichloroacetic acid, dichloroacetic acid and acetic acid. The acid diluted in a suitable solvent can be used in the step. The solvent is not specifically limited unless it is involved in the reaction, and may include, for example, methylene chloride, acetonitrile, water, a buffer wherein pH is in the range from 2 to 5 and mixtures thereof.
Examples of the "buffer solution" may include an acetate buffer. The reaction temperature in the reaction is preferably in the range of 20°C to 50°C. The reaction time for deprotection varies depending on the kind of the oligonucleic acid derivative (31), the acid and the reaction temperature, and may be in the range of 1 minute to 1 hour. The amount of the reagent to be used may be in the range of 0.8 to 100 mol per mol of the oligonucleic acid derivative attached to the solid phase support, and preferably 1 to 10 mol per mol of the compound attached to the solid support.

### (7) Step H:

Process for isolating and purifying the oligo-RNA (B) produced by Step G.
The step of isolating and purifying is a step for isolating and purifying a desired oligo-RNA from the above reaction mixture with a known method, for example, extraction, concentration, neutralization, filtration, centrifugal separation, recrystallization, reverse-phase column chromatography (C₈ to C₁₈), reverse phase cartridge column (C₈ to C₁₈), cation exchange column chromatography, anion-exchange column chromatography, gel filtration column chromatography, high performance liquid chromatography, dialysis, ultrafiltration and combinations thereof.
Examples of the "eluent" may include acetonitrile, methanol, ethanol, isopropyl alcohol, water and a mixed solvent at an arbitrary ratio.
In this case, for example, pH of the solution can be controlled to be in the range of pH 1 to 9 by adding sodium phosphate, potassium phosphate, sodium chloride, potassium chloride, ammonium acetate, triethylammonium acetate, sodium acetate, potassium acetate, tris-hydrochloric acid or ethylenediaminetetraacetic acid as an additive in a concentration of 1 mM to 2 M.

The oligoribonucleic acid (B) of desired chain length can be produced by repeating operations of Step A - Step D. In addition, in the method, the compound (23a) wherein R^{4a} is the substituent (20), the compound (23a) wherein R^{4a} is H or acyl, or the compound (23b) wherein R² is alkyloxy are used. When using the compound (23a) wherein R^{4a} is H or acyloxy or the compound (23b) wherein R² is alkyloxy as a starting material, it is necessary to use one or more units of the phosphoramidite compounds according to the present invention as a nucleic acid monomer compound.

### EXAMPLES

The present invention will now be described in more detail with reference to Examples, to which, however, the present invention is not limited.

### Reference Example 1

### Chloromethyl 2-cyanoethyl ether

### Step 1

### Production of methylthiomethyl 2-cyanoethyl ether

3-Hydroxypropionitrile (32 g, 450 mmol) was dissolved in 450 mL of dimethylsulfoxide, and 324 mL of acetic anhydride and 231 mL of acetic acid were added thereto, and the reaction solution was stirred at room temperature for 24 hours. Sodium bicarbonate (990 g) was dissolved in 4.5 L of water, and the reaction solution was added to the aqueous sodium bicarbonate solution dropwise over 1 hour, and was subjected to extraction with ethyl acetate, and the extract was dried over anhydrous magnesium sulfate, and the solvent was distilled off. The obtained oily product was purified by silica gel column chromatography to obtain 41 g of methylthiomethyl 2-cyanoethyl ether as a colorless oily product (yield 70 %).
¹H-NMR (CDCl₃): 2.18 (s, 3H), 2.66 (t, 2H, J = 6.3 Hz), 3.77 (t, 2H, J = 6.3 Hz), 4.69 (s, 2H)

### Step 2

### Production of chloromethyl 2-cyanoethyl ether

Methylthiomethyl 2-cyanoethyl ether (3.3 g, 25 mmol) was dissolved in 70 mL of methylene chloride, and 2 mL of sulfuryl chloride (25 mmol) was added dropwise, and the reaction was further performed at room temperature for 1 hour. After the reaction completed, the solvent was distilled off under reduced pressure to obtain 2.5 g of the objective compound as a colorless oily product (yield 85 %).
Boiling point: 84°C - 85°C(0.3 Torr)
¹H-NMR (CDCl₃): 2.72 (t, 2H, J = 6.3 Hz), 3.92 (t, 2H, J = 6.3 Hz), 5.52 (s, 2H)

### Reference Example 2

### 5'-O-(4,4'-dimethoxytrityl)-2'-O-(2-cyanoethoxymethyl)uridine 3'-O-(2-cyanoethyl N,N-diisopropylphosphoramidite)

### Step 1

### Production of 5'-O-(4,4'-dimethoxytrityl)-2'-O-(2-cyanoethoxymethyl)uridine

5'-O-(4,4'-Dimethoxytrityl)uridine (546 mg, 1 mmol) was dissolved in 4 mL of 1,2-dichloroethane, and 452 mg of diisopropylethylamine (3.5 mmol) was added thereto, and 365 mg of dibutylstannyl dichloride (1.2 mmol) was further added thereto. The reaction was performed at room temperature for 1 hour. Subsequently, the reaction was performed at 80°C, and 155.4 mg of chloromethyl 2-cyanoethyl ether (1.3 mmol) was added dropwise, and the reaction solution was stirred for 30 minutes. After the reaction completed, the reaction solution was added into an aqueous saturated sodium bicarbonate solution, and was subjected to extraction with methylene chloride, and the extract was dried over anhydrous magnesium sulfate, and the solvent was distilled off. The obtained mixture was purified by 30 g of silica gel column chromatography to obtain 5'-O-(4,4'-dimethoxytrityl)-2'-O-(2-cyanoethoxymethyl)uridine (197 mg, yield 34 %).
¹H-NMR (CDCl₃): 2.47 (d, 1H, J = 7.8 Hz), 2.69 (t, 2H, J = 6.3 Hz), 3.55 (dd, 1H, J = 11.3, 2.2 Hz), 3.62 (dd, 1H, J = 11.3, 2.2 Hz), 3.83 (s, 6H), 3.87 (t, 2H, J = 6.3 Hz), 4.07-4.08 (m, 1H), 4.32 (dd, 1H, J = 5.3, 1.9 Hz), 4.54 (q, 1H, J = 5.3 Hz), 4.94,5.11 (2d, 2H, J = 6.9 Hz), 5.32 (d, 1H, J = 8.2 Hz), 6.00 (d, 1H, J = 1.9 Hz), 6.85-6.88 (m, 4H), 7.29-7.41 (m, 9H), 8.02 (d, 1H, J = 8.2 Hz), 8.53 (brs, 1H) ESI-Mass: 652[M+Na]⁺

### Step 2

### Production of 5'-O-(4,4'-dimethoxytrityl)-2'-O-(2-cyanoethoxymethyl)uridine 3'-O-(2-cyanoethyl N,N-diisopropylphosphoramidite)

5'-O-(4,4'-Dimethoxytrityl)-2'-O-(2-cyanoethoxymethyl)urid ine (209 g, 0.332 mmol) was dissolved in 2 mL of acetonitrile obtained in Step 1 and 23 mg of tetrazole (0.332 mmol), and 150 mg of 2-cyanoethyl N,N,N',N'-tetraisopropylphosphorodiamidite (0.498 mmol) were added dropwise, and the reaction was performed at 45°C for 1.5 hours. After the reaction completed, the reaction solution was mixed with an aqueous saturated sodium bicarbonate solution, and was subjected to extraction with ethyl acetate, and the extract was dried over anhydrous magnesium sulfate, and the solvent was distilled off. The obtained mixture was purified by 20 g of silica gel column chromatography to obtain the objective compound (200 mg, yield 73 %).
ESI-Mass: 852[M+Na]⁺

### Reference Example 3

### 2'-O-(2-cyanoethoxymethyl)uridine

### Step 1

### Production of 3',5'-O-(tetraisopropyldisiloxane-1,3-diyl)-2'-O-(2-cyanoethoxym ethyl)uridine

3',5'-O-(Tetraisopropyldisiloxane-1,3-diyl)uridine 150 mg (0.3 mmol) was dissolved in 7 mL of THF under an argon atmosphere, and 54 mg of methylthiomethyl 2-cyanoethyl ether (0.4 mmol) and 100 mg of molecular sieves 4A were added, and the reaction solution was stirred for 10 minutes. The reaction was performed at 0°C, and 2 mL of a solution of trifluoromethanesulfonic acid (10 mg, 0.06 mmol) in THF was added. Then, 92 mg of N-iodosuccinimide (0.4 mmol) was added, and the reaction solution was stirred for 1 hour. After the reaction completed, the reaction solution was filtrated with a celite® and washed with methylene chloride, and the obtained organic layer was washed with 1 M aqueous sodium hydrogen thiosulfate solution. The organic layer was washed with aqueous saturated sodium bicarbonate solution, and dried over anhydrous magnesium sulfate, and the solvent was distilled off. The obtained residue was purified by thin-layer chromatography to obtain 3',5'-O-(tetraisopropyldisiloxan-1,3-diyl)-2'-O-(2-cyanoethoxyme thyl)uridine (150 mg, yield 85 %).
¹H-NMR (CDCl₃): 0.97-1.12 (m, 28H), 2.68-2.73 (m, 2H), 3.78-3.86 (m, 1H), 3.96-4.05 (m, 2H), 4.12-4.30 (m, 4H), 5.0-5.04 (m, 2H), 5.70 (d, 1H, J = 8.2 Hz), 5.75 (s, 1H), 7.90 (d, 1H, J = 8.2 Hz), 9.62 (brs, 1H)
ESI-Mass: 570[M+H]⁺

### Step 2

### Production of 2'-O-(2-cyanoethoxymethyl)uridine

3',5'-O-(Tetraisopropyldisiloxan-1,3-diyl)-2'-O-(2-cyanoet hoxymethyl)uridine (200 mg, 0.35 mmol) obtained in Step 1 was dissolved in 2 mL of methanol, and 65 mg of ammonium fluoride (1.76 mmol) was added thereto, and the reaction solution was stirred with heating at 50°C for 5 hours. After air-cooling, acetonitrile was added to the reaction solution. The solution was stirred, and was filtrated and concentrated. The obtained residue was purified by silica gel column chromatography to obtain the objective compound (108 mg, yield 94 %).
¹H-NMR (CD₃OD): 2.72-2.76 (t, 2H, J = 6.2 Hz), 3.68-3.92 (m, 4H), 4.00-4.03 (m, 1H), 4.26-4.32 (m, 2H), 4.81-4.95 (m, 2H), 5.71 (d, 1H, J = 8.1 Hz), 6.00 (d, 1H, J = 3.3 Hz), 8.10 (d, 1H, J = 8.1 Hz)
ESI-Mass: 350[M+Na]⁺

### Reference Example 4

### Production of 5'-O-(4,4'-dimethoxytrityl)-2'-O-(2-cyanoethoxymethyl)uridine

2'-O-(2-Cyanoethoxymethyl)uridine (14 g, 43 mmol) was subjected to azeotropic distillation with pyridine, and then was dried with a vacuum pump for 30 minutes. The residue was dissolved in 300 mL of THF, and 68 g of pyridine (856 mmol), and 20 g of molecular sieves 4A was added under an argon atmosphere, and the mixture was stirred for 10 minutes. To the solution was added 19.6 g of 4,4'-dimethoxytritylchloride (57.8 mmol) by 3 portions every 1 hour, and the mixture was further stirred for 1 hour. After 10 mL of methanol was added and the reaction solution was stirred for 2 minutes, the reaction solution was filtrated with a celite®, and was washed with ethyl acetate. After concentrating the filtrate, the residue was dissolved in ethyl acetate, and was washed with a saturated aqueous sodium bicarbonate solution. After the organic layer was washed with brine and dried over anhydrous magnesium sulfate, the solvent was distilled off. The obtained residue was purified by silica gel chromatography to obtain the objective compound (26.5 g, yield 98%).

### Reference Example 5

### N⁴-acetyl-5'-O-(4,4'-dimethoxytrityl)-2'-O-(2-cyanoethoxymethyl) cytidine 3'-O-(2-cyanoethyl N,N-diisopropylphosphoramidite)

### Step 1

### Production of N⁴-acetyl-5'-O-(4,4'-dimethoxytrityl)-2'-O-(2-cyanoethoxymethyl) Cytidine

N⁴-Acetyl-5'-O-(4,4'-dimethoxytrityl)cytidine (588 mg, 1 mmol)was dissolved in 4 mL of 1,2-dichloroethane, and 452 mg of diisopropylethylamine (3.5 mmol) was added thereto, and then 365 mg of dibutylstannyl dichloride (1.2 mmol) was further added. The reaction was performed at room temperature for 1 hour. Then, the reaction was performed at 80°C, and 155.4 mg of chloromethyl 2-cyanoethyl ether (1.3 mmol) was added dropwise, and the solution was stirred for 60 minutes. After the reaction completed, the reaction solution was added into an aqueous saturated sodium bicarbonate solution, and was extracted with methylene chloride. The extract was dried over anhydrous magnesium sulfate, and the solvent was distilled off. The obtained mixture was purified by 30 g of silica gel column chromatography to obtain N⁴-acetyl-5'-O-(4,4'-dimethoxytrityl)-2'-O-(2-cyanoethoxymethyl) cytidine (219 mg, yield 35 %).
¹H-NMR (CDCl₃): 2.19 (s, 3H), 2.56 (d, 1H, J = 8.8 Hz), 2.65 (t, 2H, J = 6.2 Hz), 3.55 (dd, 1H, J = 10.5, 2.5 Hz), 3. 63 (dd, 1H, J = 10.5, 2.5 Hz), 3.82 (s, 6H), 3.86 (t, 2H, J = 6.2 Hz), 4.09-4.14 (m, 1H), 4.28 (d, 1H, J = 5.1 Hz), 4.44-4.49 (m, 1H), 4.97,5.24 (2d, 2H, J = 6.9 Hz), 5.96 (s, 1H), 6.86-6.88 (m, 4H), 7.09 (d, 1H, J = 6.9 Hz), 7.26-7.42 (m, 9H), 8.48 (d, 1H, J = 6.9 Hz), 8.59 (brs, 1H)
ESI-Mass: 693[M+Na]⁺

### Step 2

### Production of N⁴-acetyl-5'-O-(4,4'-dimethoxytrityl)-2'-O-(2-cyanoethoxymethyl) cytidine 3'-O-(2-cyanoethyl N,N-diisopropylphosphoramidite)

N⁴-Acetyl-5'-O-(4,4'-dimethoxytrityl)-2'-O-(2-cyanoethoxym ethyl) cytidine (205 mg, 0.306 mmol) obtained in Step 1 was dissolved in 2 mL of methylene chloride, and 105 mg of diisopropylethylamine (0.812 mmol) was added, and 116 mg of 2-cyanoethyl N,N-diisopropyl chlorophosphoramidite (0.49 mmol) was added dropwise. The reaction solution was reacted at room temperature for 1 hour. After the reaction completed, the solvent was distilled off and the obtained mixture was purified by 20 g of silica gel column chromatography to obtain the objective compound (242mg, yield 91%).
ESI-Mass: 871[M+H]⁺

### Reference Example 6

### N⁴-acetyl-2'-O-(2-cyanoethoxymethyl)cytidine

### Step 1

### Production of N⁴-acetyl-3',5'-O-(tetraisopropyldisiloxane-1,3-diyl)-2'-O-(2-cy anoethoxymethyl)cytidine

N⁴-Acetyl-3',5'-O-(1,3-tetraisopropyldisiloxane-diyl)cytid ine 1.00 g (1.89 mmol) and methylthiomethyl 2-cyanoethyl ether 500 mg (3.79 mmol) were mixed, and the mixture was dissolved in mixed solvent of 10 mL of toluene and 10 mL of THF. Subsequently, 975 mg of silver trifluoromethanesulfonate was added and was dried by adding molecular sieves 4A. Under ice cooling, 370 mg of N-bromosuccinimide (2.08 mmol) was added, and the solution was stirred for 10 minutes in the reaction vessel shielded from light. Furthermore, 70 mg of N-bromosuccinimide (0.39 mmol) was added and stirred for 25 minutes. After the reaction completed, the reaction solution was diluted with methylene chloride, and was washed with an aqueous saturated sodium bicarbonate solution. The extract was dried over anhydrous sodium sulfate, and the solvent was distilled off. The obtained mixture was purified by silica gel column chromatography to obtain N'-acetyl-3',5'-O-(tetraisopropyldisiloxan-1,3-diyl)-2'-O-(2-cya noethoxymethyl)cytidine (936 mg, yield 81 %).
¹H-NMR (CDCl₃): 0.90-1.11 (m, 28H), 2.28 (s, 3H), 2.62-2.79 (m, 2H), 3.78-3.89 (m, 1H), 3.96-4.04 (m, 2H), 4.19-4.23 (m, 3H), 4.30 (d, 1H, J = 13.6 Hz), 5.00 (d, 1H, J = 6.8 Hz), 5.09 (d, 1H, J = 6.8 Hz), 5.77 (s, 1H), 7.44 (d, 1H, J = 7.5 Hz), 8.30 (d, 1H, J = 7.5 Hz), 10.13 (s, 1H)
ESI-Mass: 611[M+H]⁺

### Step 2

### Production of N⁴-acetyl-2'-O-(2-cyanoethoxymethyl)cytidine

N⁴-Acetyl-3',5'-O-(tetraisopropyldisiloxane-1,3-diyl)-2'-O -(2-cyanoethoxymethyl)cytidine (500 mg, 0.819 mmol) obtained in Step 1 was dissolved in a mixed solvent of 2.5mL of THF and 2.5 mL of methanol, and 150 mg of ammonium fluoride (4.10 mmol) was added, and then the reaction solution was reacted at 50°C for 4 hours. After the reaction completed, the reaction solution was diluted with acetonitrile and filtrated, and the solvent was distilled off. The obtained mixture was purified by silica gel column chromatography to obtain the objective compound (210 mg, yield 70 %).
¹H-NMR (D₂O): 2.13 (s, 3H), 2.66-2.71 (m, 2H), 3.72-3.78 (m, 3H), 3.90 (dd, 1H, J = 13.0, 2.6 Hz), 4.06-4.11 (m, 1H), 4.20 (dd, 1H, J = 7.1, 5.2 Hz), 4.29 (dd, 1H, J = 5.1, 2. 9 Hz), 4.83 (d, 1H, J =7.2Hz), 4. 94 (d, 1H, J = 7.2 Hz), 5.95 (d, 1H, J = 2.9 Hz), 7.25 (d, 1H, J = 7.6 Hz), 8.25 (d, 1H, J = 7.6 Hz)
ESI-Mass: 391[M+Nal⁺

### Reference Example 7

### Production of N⁴-acetyl-5'-O-(4,4'-dimethoxytrityl)-2'-O-(2-cyanoethoxymethyl) cytidine

2'-O-(2-Cyanoethoxymethyl)cytidine (9.9 g, 26.8 mmol) was subjected to azeotropic distillation with pyridine, and then was dried with a vacuum pump for 30 minutes. The residue was dissolved in 190 mL of THF, and 43 g of pyridine (538 mmol) and 20 g of molecular sieves 4A were added under an argon atmosphere, and the mixture was stirred for 10 minutes. To the reaction solution was added 11.8 g of 4,4'-dimethoxytrityl chloride 11.8 g (34.9 mmol) by 3 portions every 1 hour, and the mixture was further stirred for 1 hour. After 2 mL of methanol was added and the reaction solution was stirred for 2 minutes, the reaction solution was filtrated with a celite®, and was washed with ethyl acetate. After concentrating the filtrate with evaporation, the residue was dissolved in ethyl acetate, and was separated with a saturated aqueous sodium bicarbonate solution. After the organic layer was washed with brine and dried over anhydrous magnesium sulfate, the solvent was distilled off. The obtained residue was purified by silica gel chromatography to obtain the objective compound (15 g, yield 83 %).

### Reference Example 8

### N²-acetyl-5'-O-(4,4'-dimethoxytrityl)-2'-O-(2-cyanoethoxymethyl) guanosine 3'-O-(2-cyanoethyl N,N-diisopropylphosphoramidite)

### Step 1

### Production of N²-acetyl-5'-O-(4,4'-dimethoxytrityl)-2'-O-(2-cyanoethoxymethyl) guanosine

N²-Acetyl-5'-O-(4,4'-dimethoxytrityl)guanosine (627 mg, 1 mmol) was dissolved in 4 mL of 1,2-dichloroethane, and 452 mg of diisopropylethylamine (3.5 mmol) was added, and then 365 mg of dibutylstannyl dichloride (1.2 mmol) was added. And then, the reaction solution was reacted at room temperature for 1 hour. Then, the reaction was performed at 80°C, and 155.4 mg of chloromethyl 2-cyanoethyl ether (1.3 mmol) was added dropwise, and the solution was stirred for 60 minutes. After the reaction completed, the reaction solution was mixed with an aqueous saturated sodium bicarbonate solution, and was subjected to extraction with methylene chloride. The extract was dried over anhydrous magnesium sulfate, and the solvent was distilled off. The obtained mixture was purified by 30 g of silica gel column chromatography to obtain N²-acetyl-5'-O-(4,4'-dimethoxytrityl)-2'-O-(2-cyanoethoxymethyl) guanosine (450 mg, yield 63 %).
¹H-NMR (CDCl₃): 1.92 (s, 3H), 2.47-2.51 (m, 2H), 2.68 (brs, 1H), 3.30 (dd, 1H, J = 10.7, 3.8 Hz), 3.47 (dd, 1H, J = 10.7, 3.8 Hz), 3.55-3.60 (m, 1H), 3.65-3.70 (m, 1H), 3.74,3.75 (2s, 6H), 4.22-4.23 (m, 1H), 4.55-4.58 (m, 1H), 4.78,4.83 (2d, 2H, J = 7.0 Hz), 5.01 (t, 1H, J = 5.1 Hz), 5.99 (d, 1H, J = 5.1 Hz), 6.76-6.79 (m, 4H), 7.17-7.44 (m, 9H), 7.88 (s, 1H), 8.36 (brs, 1H), 12.06 (brs, 1H)

### Step 2

### Production of N²-acetyl-5'-O-(4,4'-dimethoxytrityl)-2'-O-(2-cyanoethoxymethyl) guanosine 3'-O-(2-cyanoethyl N,N-diisopropylphosphoramidite)

N²-Acetyl-5'-O-(4,4'-dimethoxytrityl)-2'-O-(2-cyanoethoxym ethyl) guanosine (400mg, 0.563mol) obtained in Step 1 was dissolved in 2 mL of methylene chloride, and 181 mg of diisopropylethylamine (1.4 mmol) was added, and 161 mg of 2-cyanoethyl N,N-diisopropylchloro phosphoramidite (0.68 mmol) was added dropwise. Then, the reaction was performed at room temperature for 1 hour. After the reaction completed, the solvent was distilled off and the obtained mixture was purified by 20 g of silica gel column chromatography to obtain the objective compound (471 mg, yield 92 %).

### Reference Example 9

### N⁶-acetyl-5'-O-(4,4'-dimethoxytrityl)-2'-O-(2-cyanoethoxymethyl) adenosine 3'-O-(2-cyanoethyl N,N-diisopropylphosphoramidite)

### Step 1

### Production of N⁶-acetyl-5'-O-(4,4'-dimethoxytrityl)-2'-O-(2-cyanoethoxymethyl) adenosine

N⁶-Acetyl-5'-O-(4,4'-dimethoxytrityl)adenosine (22.0 g, 36.0 mmol) was dissolved in 170 mL of 1,2-dichloroethane, and 16.3 g of diisopropylethylamine (126 mmol) was added, and 12.1 g of dibutylstannyl dichloride (39.7 mmol) was added subsequently. Then, the reaction was performed at room temperature for 1 hour. Then, the reaction solution was heated up to 80°C, and 4. 30 g of chloromethyl 2-cyanoethyl ether (36.0 mmol) was added dropwise, and the solution was stirred for 30 minutes. After the reaction completed, the reaction solution was added to an aqueous saturated sodium bicarbonate solution, and was subjected to extraction with methylene chloride. The extract was dried over anhydrous magnesium sulfate, and the solvent was distilled off. The obtained mixture was purified by silica gel column chromatography to obtain N⁶-acetyl-5'-O-(4,4'-dimethoxytrityl)-2'-O-(2-cyanoethoxymethyl) adenosine (7.47 g, yield 33 %).
¹H-NMR (CDCl₃): 2.51 (t, 2H, J = 6.2 Hz), 2.58 (d, 1H, J = 5.5 Hz), 2.61 (s, 3H), 3.45 (dd, 1H, J = 10.7, 4.0 Hz), 3.54 (dd, 1H, J = 10.7, 3.2 Hz), 3.62-3.79 (m, 2H), 3.79 (s, 6H), 4.25 (brq, 1H, J = 4.6 Hz), 4.59 (q, 1H, J = 5.2 Hz), 4.87-4.94 (m, 3H), 6.23 (d, 1H, J = 4.4 Hz), 6.80-6.83 (m, 4H), 7.22-7.32 (m, 7H), 7.40-7.43 (m, 2H), 8.20 (s, 1H), 8.61 (brs, 1H), 8.62 (s, 1H)
ESI-Mass: 695[M+H]⁺

### Step 2

### Production of N⁶-acetyl-5'-O-(4,4'-dimethoxytrityl)-2'-O-(2-cyanoethoxymethyl) adenosine 3'-O-(2-cyanoethyl N,N-diisopropylphosphoramidite)

N⁶-Acetyl-5'-O-(4,4'-dimethoxytrityl)-2'-O-(2-cyanoethoxym ethyl)adenosine (10.0 g, 14.4 mmol) obtained in Step 1 was dissolved in 75 mL of methylene chloride, and 4.7 g of diisopropylethylamine (36 mmol) was added, and 4.82 g of 2-cyanoethyl N,N-diisopropylchloro phosphoramidite (20.3 mmol) was added dropwise. Then, the reaction was performed at room temperature for 1 hour. After the reaction completed, the solvent was distilled off and the obtained mixture, in which about 30 mL of the solvent remained, was purified by silica gel column chromatography to obtain the objective compound (12.0 g, yield 93 %).
ESI-Mass: 895[M+H]⁺

### Reference Example 10

### N⁶-acetyl-2'-O-(2-cyanoethoxymethyl)adenosine

### Step 1

### Production of N⁶-acetyl-3',5'-O-(tetraisopropyldisiloxan-1,3-diyl)-2'-O-(2-cya noethoxymethyl)adenosine

To 8mL of methylene chloride was suspended 245 mg of N-iodosuccinimide (1.09 mmol) and 280 mg of silver trifluoromethanesulfonate (1.09 mmol), and the solution was dried by adding molecular sieves 4A. To the reaction solution was added a solution of N⁶-acetyl-3',5'-O-(tetraisopropyldisiloxane-1,3-diyl)adenosine (400 mg, 0.73 mmol) and 145 mg of methylthiomethyl 2-cyanoethyl ether (1.11 mmol) in 4 mL of methylene chloride under ice cooling, and the reaction mixture was stirred for 3 hours. After the reaction completed, the reaction mixture was diluted with methylene chloride, and was washed with aqueous sodium thiosulfate solution and aqueous saturated sodium bicarbonate solution. The extract was dried over anhydrous magnesium sulfate, and the solvent was distilled off. The obtained mixture was purified by silica gel column chromatography to obtain N⁶-acetyl-3',5'-O-(tetraisopropyldisiloxan-1,3-diyl)-2'-O-(2-cya noethoxymethyl)adenosine (201 mg, yield 45 %).
¹H-NMR (CDCl₃) : 0.98-1.11 (m, 28H), 2.62 (s, 3H), 2.69 (td, 2H, 6.5, J = 1.5Hz), 3.81-3.89 (m, 1H), 4.02-4.09 (m, 2H), 4.17 (d, 1H, J=9.4Hz), 4.28 (d, 1H, J = 13.4 Hz), 4.50 (d, 1H, J = 4.5 Hz), 4.67 (dd, 1H, J = 8.8, 4.5 Hz), 5.02 (d, 1H, J = 7.0 Hz), 5.08 (d, 1H, J = 7.0 Hz), 6.10 (s, 1H), 8.34 (s, 1H), 8.66 (s, 1H), 8.67 (s, 1H)
ESI-Mass: 636[M+H]⁺

### Step 2

### Production of N⁶-acetyl-2'-O-(2-cyanoethoxymethyl)adenosine

N⁶-Acetyl-3',5'-O-(tetraisopropyldisiloxane-1,3-diyl)-2'-O -(2-cyanoethoxymethyl)adenosine (300 mg, 0.47 mmol) obtained in Step 1 was dissolved in a mixed solvent of 0.1 mL of acetic acid and 2 mL of 0. 5 M TBAF / THF solution, and the reaction solution was stirred at room temperature for 2 hours. After the reaction completed, the obtained reaction mixture was purified by silica gel column chromatography to obtain the objective compound (160 mg, yield 86%).
¹H-NMR (DMSO-d₆): 2.25 (s, 3H), 2.53-2.68 (m, 2H), 3.41-3.46 (m, 1H), 3.56-3.64 (m, 2H), 3.69-3.73 (m, 1H), 4.00-4.01 (m, 1H), 4.36-4.37 (m, 1H), 4.72-4.78 (m, 3H), 5.20 (bt, 2H), 5.41 (d, 1H, J = 5.2 Hz), 6.17 (d, 1H, J = 5.7 Hz), 8.66 (s, 1H), 8.72 (s, 1H), 10.72 (s, 1H)
ESI-Mass: 415[M+Na]⁺

### Reference Example 11

### Production of N⁶-acetyl-2'-O-(2-cyanoethoxymethyl)adenosine

N⁶-Acetyl-2'-O-(2-cyanoethoxymethyl)adenosine (9.50 g, 24.2 mmol) was dissolved in 100 mL of dehydrated pyridine, and then was dried by concentration. Then, the residue was dissolved in 100 mL of dehydrated pyridine under an argon atmosphere. Under ice cooling, 10.7 g of 4,4'-dimethoxytrityl chloride (31.2 mmol) was added, and the reaction was performed at room temperature for 1 hour and 20 minutes. After the reaction completed, the reaction solution was diluted with methylene chloride, and was washed with water. The extract was dried over anhydrous sodium sulfate, and the solvent was distilled off. The obtained mixture was purified by silica gel column chromatography to obtain the objective compound (13.8 g, yield 82 %).

### Reference Example 12

### N²-phenoxyacetyl-5'-O-(4,4'-dimethoxytrityl)-2'-O-(2-cyanoethoxy methyl)guanosine 3'-O-(2-cyanoethyl N,N-diisopropylphosphoramidite)

### Step 1

### Production of N²-phenoxyacetyl-5'-O-(4,4'-dimethoxytrityl)-2'-O-(2-cyanoethoxy methyl)guanosine

N²-Phenoxyacetyl-5'-O-(4,4'-dimethoxytrityl)guanosine (720 mg, 1 mmol) was dissolved in 4 mL of 1,2-dichloroethane, and 452 mg of diisopropylethylamine (3.5 mmol) was added, and 365 mg of dibutylstannyl dichloride (1.2 mmol) was added subsequently. Then, the reaction was performed at room temperature for 1 hour. Then, the reaction was performed at 80°C, and 155.4 mg of chloromethyl 2-cyanoethyl ether (1.3 mmol) was added dropwise, and the solution was stirred for 60 minutes. After the reaction completed, the reaction solution was mixed with an aqueous saturated sodium bicarbonate solution, and was subjected to extraction with methylene chloride. The extract was dried over anhydrous magnesium sulfate, and the solvent was distilled off. The obtained mixture was purified by 30 g of silica gel column chromatography to obtain N²-phenoxyacetyl-5'-O-(4,4'-dimethoxytrityl)-2'-O-(2-cyanoethoxy methyl)guanosine (384 mg, yield 48 %).
¹H-NMR (CDCl₃): 2.47-2.51 (m, 2H), 2.58 (brs, 1H), 3.42 (dd, 1H, J = 10.1, 3.8 Hz), 3.46 (dd, 1H, J = 10.1, 3.8 Hz), 3.53-3.57 (m, 1H), 3.69-3.73 (m, 1H), 3.77 (s, 6H), 4.24-4.26 (m, 1H), 4.48-4.50 (m, 1H), 4.61-4.65 (m, 2H), 4.83,4.87 (2d, 2H, J = 7.0 Hz), 4.88 (t, 1H, J =5.7Hz), 6.05 (d, 1H, J = 5.7 Hz), 6.80-6.82 (m, 4H), 6.92-6.96 (m, 3H), 7.07-7.11 (m, 2H), 7.20-7.42 (m, 9H), 7.84 (s, 1H), 8.99 (s, 1H), 11.81 (brs, 1H)
ESI-Mass: 825[M+Na]⁺

### Step 2

### Production of N²-phenoxyacetyl-5'-O-(4,4'-dimethoxytrityl)-2'-O-(2-cyanoethoxymethyl)guanosine 3'-O-(2-cyanoethyl N,N-diisopropylphosphoramidite)

N²-Phenoxyacetyl-5'-O-(4,4'-dimethoxytrityl)-2'-O-(2-cyanoethoxy methyl) guanosine (320 mg, 0.399 mmol) obtained in Step 1 was dissolved in 4 mL of methylene chloride, and 128.8 mg of diisopropylethylamine (0.996 mmol) was added, and 141.5 mg of 2-cyanoethyl N,N-diisopropylchlorophosphoramidite (0.598 mmol) was added dropwise. Then, the reaction was performed at room temperature for 1 hour. After the reaction completed, the solvent was distilled off and the obtained mixture was purified by 30 g of silica gel column chromatography to obtain the objective compound (316 mg, yield 79 %).
ESI-Mass: 1,003[M+H]⁺

### Reference Example 13

### N²-phenoxyacetyl-2'-O-(2-cyanoethoxymethyl)guanosine

### Step 1

### Production of N²-phenoxyacetyl-3',5'-O-(tetraisopropyldisiloxane-1,3-diyl)-2'-O-(2-cyanoethoxymethyl)guanosine

N²-Phenoxyacetyl-3',5'-O-(1,3-tetraisopropyldisiloxane-1,3 -diyl) guanosine (2.0 g, 3.0 mmol) was dissolved in 16 mL of THF, and 0.99 g of methylthiomethyl 2-cyanoethyl ether (7.6 mmol) and 1.0 g of molecular sieves 4A were added, and the reaction solution was stirred at -45°C for 10 minutes under an argon atmosphere. After a solution of 0.68 g of trifluoromethanesulfonic acid (4.5 mmol) in 5 mL of THF was added and the reaction solution was stirred, 1.02 g of N-iodosuccinimide (4.5 mmol) are added, and the reaction solution was stirred for 15 minutes. After saturated aqueous sodium bicarbonate solution was added to the reaction solution and then the reaction solution was filtrated, the organic layer was washed with 1 M aqueous sodium hydrogen thiosulfate solution. Further, the reaction solution was washed with water and saturated brine sequentially, and the extract was dried over anhydrous magnesium sulfate, and the solvent was distilled off. The obtained residue was purified by silica gel chromatography to obtain N²-phenoxyacetyl-3',5'-O-(tetraisopropyldisiloxan-1,3-diyl)-2'-O -(2-cyanoethoxymethyl)guanosine (2.0 g, yield 89 %).
¹H-NMR (CDCl₃): 0.99-1.11 (m, 28H), 2.59-2.77 (m, 2H), 3.82-4.05 (m, 3H), 4.15 (d, 1H, J = 9.3 Hz), 4.25-4.35 (m, 2H), 4.52-4.56 (dd, 1H, J=9.3, 4.3Hz), 5.00, 5.07 (2d, 2H, J = 7.2 Hz), 5.95 (s, 1H) 6.99-7.12 (m, 3H), 7.35-7.40 (m, 2H), 8.09 (s, 1H), 9.38 (brs, 1H), 11.85 (brs, 1H)
ESI-Mass: 766[M+Na]⁺

### Step 2

### Production of N²-phenoxyacetyl-2'-O-(2-cyanoethoxymethyl)guanosine

The solution consisting of 0.14 mL of acetic acid (0.14 mmol) and 2.83 mL of 1M TBAF in THF (2.83 mmol) was prepared. N²-Phenoxyacetyl-3'5'-O-(tetraisopropyldisiloxane-1,3-diyl)-2'-O -(2-cyanoethoxymethyl)guanosine 1.0 g (1.35 mmol) obtained in Step 1 was dissolved in 2.83 mL of THF, and the solution prepared above was added, and the reaction was performed at room temperature for 1 hour under an argon atmosphere. The reaction solution was concentrated under reduced pressure, and the residue was dissolved in methylene chloride, and was purified by silica gel column chromatography to obtain the objective compound (0.67 g, yield 99%).
¹H-NMR (DMSO-d₆): 2.59-2.66 (m, 2H), 3.41-3.63 (m, 4H), 3.98 (m, 1H), 4.32 (m, 1H), 4.58-4.62 (t, 1H, J = 5.3 Hz), 4.71-4.78 (dd, 2H, J = 13.1, J = 6.8 Hz), 4.87 (s, 2H), 5.12 (s, 1H) 5.37 (s, 1H), 5.97 (d, 1H, J = 6.1 Hz) 6.96-6.99 (m, 3H), 7.28-7.34 (m, 2H), 8.30 (s, 1H), 11.78 (brs, 2H)
ESI-Mass: 500[M-H]⁻

### Reference Example 14

### Production of N²-phenoxyacetyl-5'-O-(4,4'-dimethoxytrityl)-2'-O-(2-cyanoethoxymethyl)guanosine

N²-Phenoxyacetyl-2'-O-(2-cyanoethoxymethyl)guanosine (660 mg, 1.32 mmol) was subjected to azeotropic distillation with pyridine, and then was dried with a vacuum pump for 30 minutes. The residue was dissolved in 9 mL of THF, and 2.1 g of pyridine (26.4mmol) and 600 mg of molecular sieves 4A were added under an argon atmosphere, and the reaction solution was stirred for 10 minutes. To the solution was added 540 mg of 4,4'-dimethoxytritylchloride (1.58 mmol) by 3 portions every 1 hour, and the reaction solution was further stirred for 1 hour. After 2 mL of methanol was added and the reaction solution was stirred for 2 minutes, the reaction solution was filtrated with a celite®, and was washed with ethyl acetate. After concentrating the filtrate with evaporation, the residue was dissolved in ethyl acetate, and was separated with a saturated aqueous sodium bicarbonate solution. After the organic layer was washed with a saturated brine and dried over anhydrous magnesium sulfate, the solvent was distilled off. The obtained residue was purified by silica gel chromatography to obtain the objective compound (800 mg, yield 75%).

### Reference Example 15

### N⁶-acetyl-3',5'-O-(tetraisopropyldisiloxane-1,3-diyl)-2'-O-(2-cy anoethoxymethyl)adenosine

### Step 1

### Production of N⁶-acetyl-3',5'-O-(tetraisopropyldisiloxane-1,3-diyl)-2'-O-methy lthiomethyl adenosine

N⁶-Acetyl-3',5'-O-(tetraisopropyldisiloxan-1,3-diyl)adenos ine (2.00 g, 3.62 mmol) was dissolved in 25 mL of dimethylsulfoxide, and 17.5 mL of acetic anhydride and 12.5 mL of acetic acid were added, and the reaction solution was stirred at room temperature for 14 hours. After the reaction completed, the reaction solution was added to 200 mL of water, extracted with ethyl acetate, and was washed with saturated aqueous sodium bicarbonate solution. The extract was dried over anhydrous sodium sulfate, and the solvent was distilled off. The obtained mixture was purified by silica gel column chromatography to obtain N⁶-acetyl-3',5'-O-(tetraisopropyldisiloxan-1,3-diyl)-2'-O-methyl thiomethyl adenosine (1.36 g, yield 61%).
¹H-NMR (CDCl₃): 0.96-1.11 (m, 28H), 2.20 (s, 3H), 2.61 (s, 3H), 4.03 (dd, 1H, J = 13.4, 2.4 Hz), 4.18 (d, 1H, J = 9.1 Hz), 4.27 (d, 1H, J = 13.4 Hz), 4.63-4.71 (m, 2H), 5.00 (d, 1H, J = 11.5 Hz), 5.07 (d, 1H, J = 11.5 Hz), 6.09 (s, 1H), 8.31 (s, 1H), 8.65 (s, 1H), 8.69 (s, 1H)
ESI-Mass: 635[M+Na]⁺

### Step 2

### Production of N⁶-acetyl-3',5'-O-(tetraisopropyldisiloxane-1,3-diyl)-2'-O-(2-cy anoethoxymethyl)adenosine

N⁶-Acetyl-3',5'-O-(tetraisopropyldisiloxane-1,3-diyl)-2'-O -methylthiomethyl adenosine (1.00 g, 1.63 mmol) obtained in Step 1 was dissolved in 25 mL of THF. To the reaction solution was added 5.88 g of 3-hydroxypropionitrile (82.7 mmol), and the solution was dried by adding molecular sieves 4A, and was cooled to -45°C. To the reaction solution were added 440 mg of N-iodosuccinimide (1.96 mmol) and then 490 mg of trifluoromethanesulfonic acid (3.26 mmol), and the reaction solution was stirred at -45°C for 15 minutes. After the reaction completed, the reaction solution was neutralized by adding triethylamine while cooling, and diluted with methylene chloride. The reaction solution was washed with aqueous sodium thiosulfate solution and saturated aqueous sodium bicarbonate solution, the extract was dried over anhydrous sodium sulfate, and the solvent was distilled off. The obtained mixture was purified by silica gel column chromatography to obtain the objective compound (722 mg, yield 71%).

### Reference Example 16

### Production of cytidylyl-[3'→5']-uridinyl-[3'→5']-uridinyl-[3'→5']-adenylyl-[3' →5']-cytidylyl-[3'→5']-guanylyl-[3'→5']-cytidylyl-[3'→5']-uridin yl-[3'→5']-guanylyl-[3'→5']-adenylyl-[3'→5']-guanylyl-[3'→5']-ur idinyl-[3'→5']-adenylyl-[3'→5']-cytidylyl-[3'→5']-uridinyl-[3'→5 ']-uridinyl-[3'→5']-cytidylyl-[3'→5']-guanylyl-[3'→5']-adenylyl-[3'→5']-uridine

The oligo-RNA of the title compound was synthesized by putting commercially available CPG solid support (37 mg, 1 µmol) containing 2'/3'-O-benzoyl-5'-O-(4,4'-dimethoxytrityl)uridine into a column with a glass filter and using an automatic nucleic acid synthesizer (Expedite^{™}: Applied Biosystems). 5'-O-(4,4'-dimethoxytrityl)-2'-O-(2-cyanoethoxymethyl)uridine 3'-O-(2-cyanoethyl N,N-diisopropylphosphoramidite), N⁴-acetyl-5'-O-(4,4'-dimethoxytrityl)-2'-O-(2-cyanoethoxymethyl) cytidine 3'-O-(2-cyanoethyl N,N-diisopropylphosphoramidite), N⁶-acetyl-5'-O-(4,4'-dimethoxytrityl)-2'-O-(2-cyanoethoxymethyl) adenosine 3'-O-(2-cyanoethyl N,N-diisopropylphosphoramidite) and N²-phenoxyacetyl-5'-O-(4,4'-dimethoxytrityl)-2'-O-(2-cyanoethoxy methyl)guanosine 3'-O-(2-cyanoethyl N,N-diisopropylphosphoramidite) as a nucleic acid monomer compound; 5-ethylthiotetrazole as a condensation catalyst; iodine solution as an oxidizing agent; phenoxyacetic anhydride and N-methylimidazole solution as a capping solution were used. After condensing nucleic acid monomer compounds 19 times, the 5'-end hydroxyl protecting group was removed on the solid phase. Then, the oligo-RNA was cleaved by reacting with concentrated aqueous ammonia - ethanol mixture (3:1) as an cleaving agent at 40°C for 4 hours, and the protecting groups of each phosphate and base were removed. After concentrating the reaction mixture under reduced pressure, the residue was reacted with THF solution of 1M TBAF containing 10 % n-propylamine and 0.6% 2-mercaptoethyl ether at room temperature for 1 hour to removed the 2'-hydroxyl protecting group. After desalting the reaction solution, the reaction solution was purified with DEAE-ion exchange resin (TOYOPEARL DEAE-650) to obtain the high purity objective compound (112 OD₂₆₀, yield 58%).
Here, absorbance of ultraviolet at wavelength 260 nm (OD₂₆₀) shows a yield of an objective compound.
Hereinafter, absorbance (OD₂₆₀) means a yield of an objective compound.
MALDI-TOF-MS:
Calculated: 6,305.9[M+H]⁺
Observed: 6,304.8[M+H]⁺

### Test Example 1

### N⁴-acetyl-2'-O-(2-cyanoethoxymethyl) cytidine

50 g (95 mmol) of N⁴-acetyl-3',5'-O-(tetraisopropyl disiloxan-1,3-diyl)cytidine was dissolved in 500 mL (142 mmol) of THF, and 18.64 g of 2-cyanoethyl methylthiomethyl ether and 40 g of molecular sieves 4A were added thereto, and then, the resulting mixture was stirred under an argon atmosphere at -45°C for 30 minutes. After 21.41 g (142 mmol) of trifluoromethane sulfonic acid was added dropwise thereto, 31.97 g (142 mmol) of N-iodosuccinimide was added thereto and the resulting mixture was stirred for 30 minutes. To the reaction mixture, 80 mL of triethyleneamine was added, followed by filtration. Then, the resulting filtrate was extracted with ethyl acetate, and the resulting organic layer was washed with a 1 M aqueous sodium thiosulfate solution, an aqueous saturated sodium bicarbonate solution and an aqueous saturated sodium chloride solution. The washed organic layer was dried over anhydrous sodium sulfate, and the solvent was distilled off.
The obtained residue was dissolved in 300 mL of THF, and 18.3 g (110 mmol) of triethylamine trihydrofluoride was added thereto, and then, the resulting mixture was stirred at 45°C for 2 hours. The deposited precipitate was collected by suction filtration, washed with cooled THF and then dried, whereby a desired compound was obtained (27 g, yield: 78%).
ESI-Mass: 391.3 [M+Na]⁺

**[TABLE1]**

| | Results |
|---|---|
| Test example 1 | A desired compound was obtained as a precipitate without performing purification using a silica gel column. |
| Reference example 6 | A desired compound was obtained by performing purification using a silica gel column. |

In the case of a cytidine derivative in which the 2'-hydroxyl group of a ribose was protected with 1- (2-cyanoethoxy) ethyl and the 3'-hydroxyl group and the 5'-hydroxyl group of the ribose were protected with disiloxyl, when the disiloxyl which protected the 3'-hydroxyl group and the 5'-hydroxyl group were removed by using triethylamine trihydrofluoride, a desired compound could be obtained as a precipitate without performing silica gel column purification.

### Test Example 2

### N²-phenoxyacetyl-2'-O-(2-cyanoethoxy methyl)guanosine

47 g (63 mmol) of N²-phenoxyacetyl-3',5'-O-(tetraisopropyldisiloxan-1,3-diyl)-2'-O -(2-cyanoethoxymethyl)guanosine was dissolved in 280 mL of acetonitrile, and 15.3 g (95 mmol) of triethylamine trihydrofluoride was added thereto, and then, the resulting mixture was stirred at 35°C for 2 hours. The reaction mixture was extracted twice with 100 mL of hexane, and 30 mL of water was added to the remaining acetonitrile layer, and then, the resulting mixture was stirred at room temperature for 5 minutes. The deposited precipitate was collected by suction filtration, washed with a cooled mixed solvent (water : acetonitrile = 1 : 1) and then dried, whereby a desired compound was obtained (22 g, yield: 69%).
ESI-Mass: 500 [M-H]⁻

**[TABLE2]**

| | Results |
|---|---|
| Test example 2 | A desired compound was obtained as a precipitate without performing purification using a silica gel column. |
| Reference example 13 | A desired compound was obtained by performing purification using a silica gel column. |

In the case of a guanosine derivative in which the 2'-hydroxyl group of a ribose was protected with 1- (2-cyanoethoxy) ethyl and the 3'-hydroxyl group and the 5'-hydroxyl group of the ribose were protected with disiloxyl, when the disiloxyl which protected the 3'-hydroxyl group and the 5'-hydroxyl group were removed by using triethylamine trihydrofluoride, a desired compound could be obtained as a precipitate without performing silica gel column purification.

### Test Example 3

### N⁶-acetyl-2'-O-(2-cyanoethoxymethyl) adenosine

44 g (69 mmol) of N⁶-acetyl-3',5'-O-(tetraisopropyldisiloxan-1,3-diyl)-2'-O-(2-cya noethoxymethyl)adenosine was dissolved in 150 mL of THF, and a solution prepared by dissolving 13.4 g (83 mmol) of triethylamine trihydrofluoride in 50 mL of THF was added thereto, and then, the resulting mixture was stirred at 45°C for 1 hour. After completion of the reaction, 50 mL of hexane was added thereto, and the resulting mixture was stirred under ice cooling. The deposited precipitate was collected by suction filtration, and a desired compound was obtained (29 g, quantitative).
ESI-Mass: 415.4 [M+Na]⁺

**[TABLE3]**

| | Results |
|---|---|
| Test example 3 | A desired compound was obtained as a precipitate without performing purification using a silica gel column. |
| Reference example 10 | A desired compound was obtained by performing purification using a silica gel column. |

In the case of an adenosine derivative in which the 2'-hydroxyl group of a ribose was protected with 1- (2-cyanoethoxy) ethyl and the 3'-hydroxyl group and the 5'-hydroxyl group of the ribose were protected with disiloxyl, when the disiloxyl which protected the 3'-hydroxyl group and the 5'-hydroxyl group were removed by using triethylamine trihydrofluoride, a desired compound could be obtained as a precipitate without performing silica gel column purification.

### [Industrial applicability]

According to the present invention, a ribonucleic acid derivative (3) can be obtained as a precipitate at a low cost and further with a high purity without performing a purification procedure using a silica gel column by using a salt of a tertiary amine with hydrofluoric acid or a mixture of a tertiary amine and hydrofluoric acid in the step of removing silicon substituents which protect the 3'-hydroxyl group and the 5'-hydroxyl group of a ribose of a ribonucleic acid derivative (1).
Thus, according to the present invention, it is possible to produce at a low cost a phosphoramidite compound (A) which can be used in the production of an oligo-RNA (B).

## Claims

1. A method for producing a ribonucleic acid derivative represented by the following general formula (3), **characterized by** allowing a salt of a tertiary amine with hydrofluoric acid represented by the following general formula (2) or a mixture of the tertiary amine and hydrofluoric acid to act on a ribonucleic acid derivative represented by the following general formula (1), thereby removing silicon substituent which protect the 3'-hydroxyl group and the 5'-hydroxyl group of a ribose thereof. 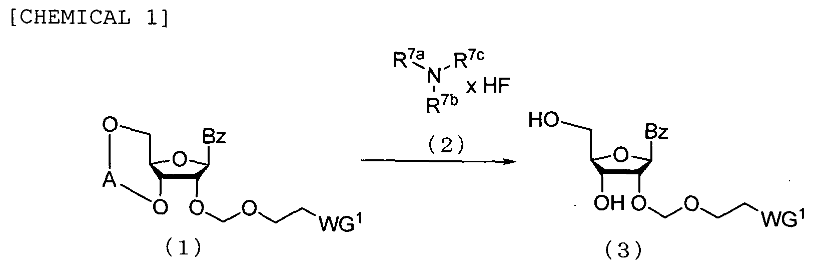 In the general formulae (1), (2) and (3), B_{z} represents a nucleobase which may have protecting groups, or a modified form thereof. R^{7a}, R^{7b} and R^{7c} are the same or different from one another and represent alkyl; or R^{7a}, R^{7b} and R^{7c} represent a saturated bicyclic amino group when combined together with the adjacent nitrogen atom. x represents an integer in the range of 1 to 30. WG¹ represents an electron-withdrawing group. A represents a silicon substituent represented by the following general formula (4a) or (4b). 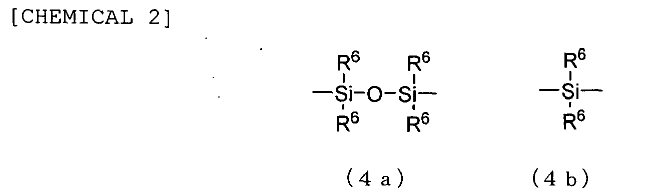 In the general formulae (4a) and (4b), R⁶ represents alkyl.

2. The method for producing a ribonucleic acid derivative according to claim 1, wherein x is an integer in the range of 2 to 15.

3. The method for producing a ribonucleic acid derivative according to either of claims 1 and 2, wherein the salt of a tertiary amine with hydrofluoric acid is triethylamine trihydrofluoride.

4. The method for producing a ribonucleic acid derivative according to any one of claims 1 to 3, wherein WG¹ is cyano.

5. The method for producing a phosphoramidite compound represented by the following general formula (A), **characterized by** including the following step. 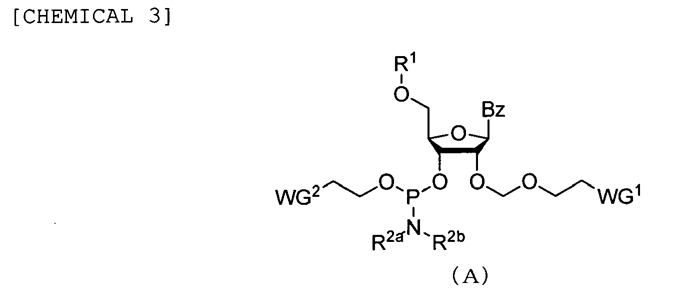 In the general formula (A), B_{z} represents a nucleobase which may have protecting groups, or a modified form thereof. R¹ represents a substituent represented by the following general formula (5). 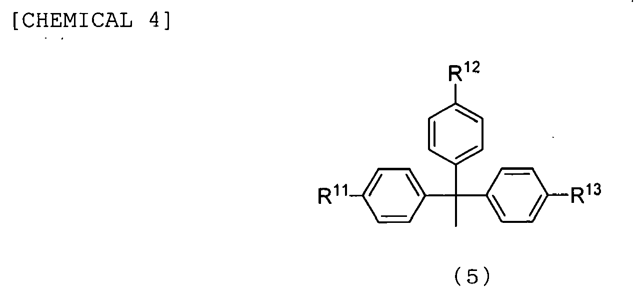 In the general formula (5), R¹¹, R¹² and R¹³ are the same or different and each represents hydrogen or alkoxy. R^{2a} and R^{2b} are the same or different and represent alkyl; or R^{2a} and R^{2b} represent a 5- or 6-membered saturated cyclic amino group when combined together with the adjacent nitrogen atom. The saturated cyclic amino group may have one oxygen atom or one sulfur atom as a ring-forming atom in addition to the nitrogen atom. WG¹ and WG² are the same or different and each represents an electron-withdrawing group.
Step:
Process for producing a ribonucleic acid derivative represented by the following general formula (3), **characterized by** allowing a salt of a tertiary amine with hydrofluoric acid represented by the following general formula (2) or a mixture of the tertiary amine and hydrofluoric acid to act on a ribonucleic acid derivative represented by the following general formula (1), thereby removing silicon substituent which protects the 3'-hydroxyl group and the 5'-hydroxyl group of a ribose 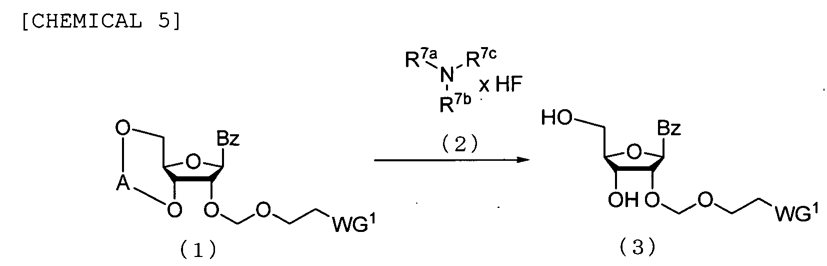 In the general formulae (1), (2) and (3), B_{Z} and WG¹ have the same meanings as above. R^{7a}, R^{7b} and R^{7c} are the same or different from one another and represent alkyl; or R^{7a}, R^{7b} and R^{7c} represent a saturated bicyclic amino group when combined together with the adjacent nitrogen atom. x represents an integer in the range of 1 to 30. A represents a silicon substituent represented by the following general formula (4a) or (4b). 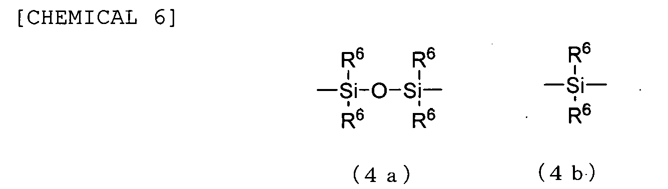 In the general formulae (4a) and (4b), R⁶ represents alkyl.

6. The method for producing a phosphoramidite compound according to claim 5, wherein x represents an integer in the range of 2 to 15.

7. The method for producing a phosphoramidite compound according to either of claims 5 and 6, wherein the salt of a tertiary amine with hydrofluoric acid is triethylamine trihydrofluoride.

8. The method for producing a phosphoramidite compound according to any one of claims 5 to 7, wherein WG¹ is cyano.

9. The method for producing a phosphoramidite compound represented by the following general formula (A), **characterized by** including following Steps a to f. 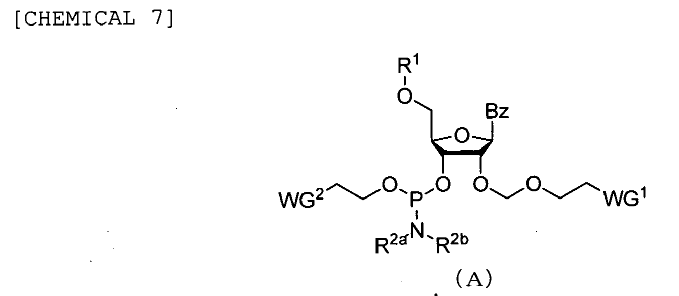 In the general formula (A), B_{Z} represents a nucleobase which may have protecting groups, or a modified form thereof. R¹ represents a substituent represented by the following general formula (5). 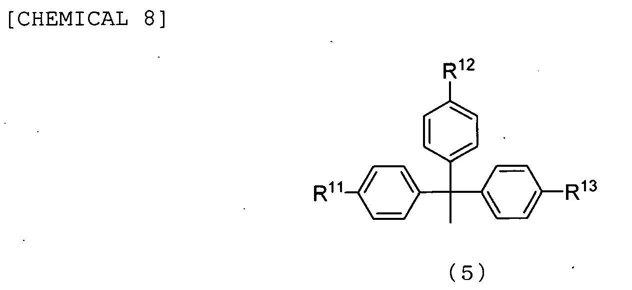 In the general formula (5), R¹¹, R¹² and R¹³ are the same or different and each represents hydrogen or alkoxy. R^{2a} and R^{2b} are the same or different and represent alkyl; or R^{2a} and R^{2b} represent a 5- or 6-membered saturated cyclic amino group when combined together with the adjacent nitrogen atom. The saturated cyclic amino group may have one oxygen atom or one sulfur atom as a ring-forming atom in addition to the nitrogen atom. WG¹ and WG² are the same or different and each represents an electron-withdrawing group.
Step a:
Process for producing a nucleoside derivative represented by the following general formula (6), wherein an ether-type protecting group which can be removed under neutral conditions is introduced at 2'-hydroxyl position, by allowing an alkylating reagent to act on a nucleoside derivative represented by the following general formula (1), 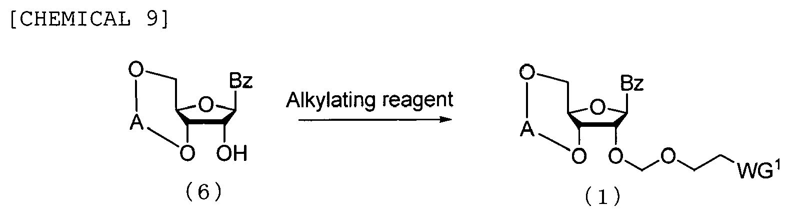 In the general formulae (1) and (6), B_{Z} and WG¹ have the same meanings as above. A represents a silicon substituent represented by the following general formula (4a) or (4b). 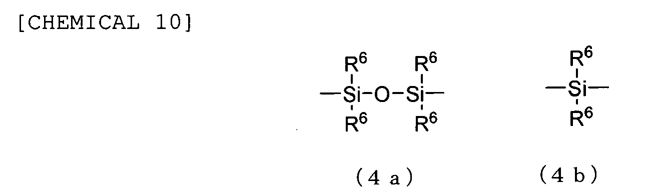 In the general formulae (4a) and (4b), R⁶ represents alkyl.
Step b:
Process for producing a ribonucleic acid derivative represented by the following general formula (7) by allowing dimethylsulfoxide, acetic acid and acetic anhydride to act on the ribonucleic acid derivative (6), being independent of Step a. 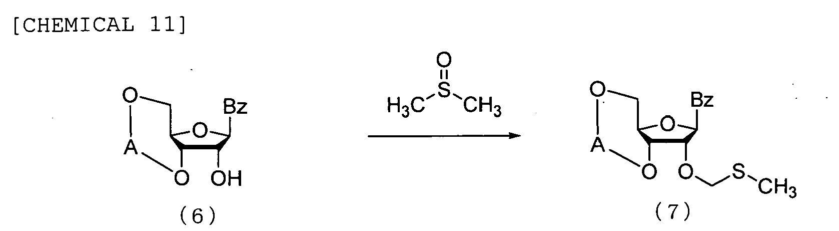 In the general formulae (6) and (7), A and B_{z} have the same meanings as above.
Step c:
Process for producing a ribonucleic acid derivative represented by the following general formula (1), wherein an ether-type protecting group which can be removed under neutral conditions is introduced at the 2'-hydroxyl position, by allowing an alcohol compound represented by the following general formula (8), an acid and a reagent for halogenating a sulfur atom to act on a ribonucleic acid derivative (7) produced in Step b, 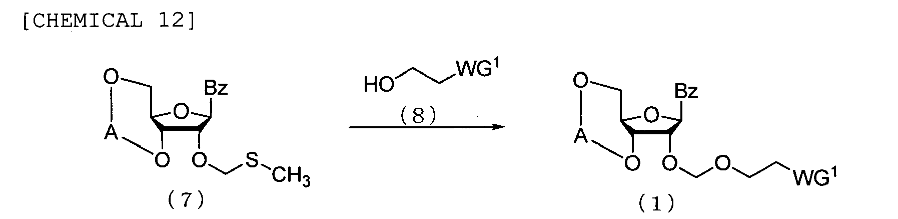 In the general formulae (1), (7) and (8), A, B_{Z} and WG¹ have the same meanings as above.
Step d:
Process for producing a ribonucleic acid derivative represented by the following general formula (3), **characterized by** allowing a salt of a tertiary amine with hydrofluoric acid represented by the following general formula (2) or a mixture of the tertiary amine and hydrofluoric acid to act on a ribonucleic acid derivative represented by the following general formula (1), thereby removing the silicon substituent which protects the 3'-hydroxyl group and the 5'-hydroxyl group of a ribose. 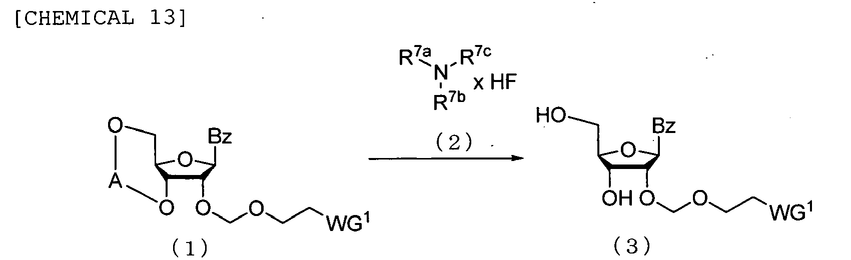 In the general formulae (1), (2) and (3), A, B_{z} and WG¹ have the same meanings as above. R^{7a}, R^{7b} and R^{7c} are the same or different from one another and represent alkyl; or R^{7a}, R^{7b} and R^{7c} represent a saturated bicyclic amino group when combined together with the adjacent nitrogen atom. x represents an integer in the range of 1 to 30.
Step e:
Process for producing a ribonucleic acid derivative (10) by introducing a protecting group (R¹), which can be removed under acidic conditions, to the 5'-hydroxyl group of the ribonucleic acid derivative (3) produced in Step d. 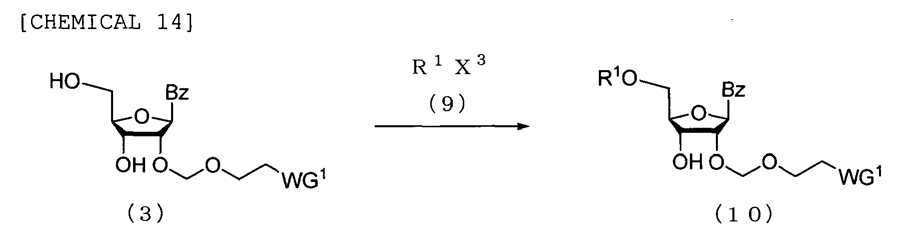 In the general formulae (3), (9) and (10), B_{z}, R¹ and WG¹ have the same meanings as above. X³ represents halogen.
Step f:
Process for producing a phosphoramidite compounds represented by the following general formula (A), wherein the 3'-hydroxyl group is phosphoramidited, by allowing a phosphoramiditing reagent and if necessary an activating agent to act on the ribonucleic acid derivative (10) produced in Step e. 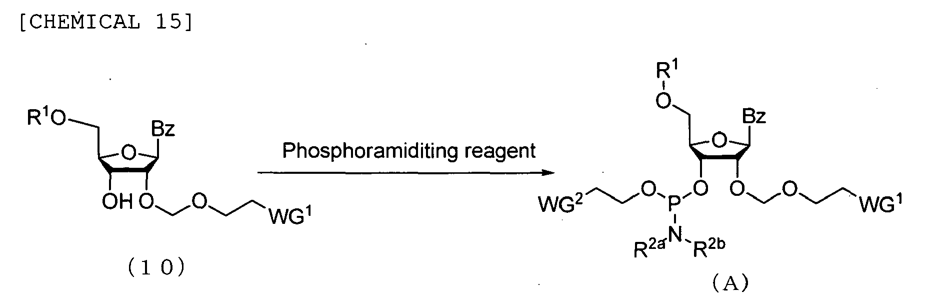 In the general formulae (10) and (A), B_{z}, R¹ and WG¹ have the same meanings as above. R^{2a} and R^{2b} are the same or different and represent alkyl; or R^{2a} and R^{2b} represent a 5- or 6-membered saturated cyclic amino group when combined together with the adjacent nitrogen atom. The saturated cyclic amino group may have one oxygen atom or one sulfur atom as a ring-forming atom in addition to the nitrogen atom. WG² represents an electron-withdrawing group.

10. The method for producing a phosphoramidite compound according to claim 9, wherein an alkylating agent is an ether compound represented by the following general formula (11). 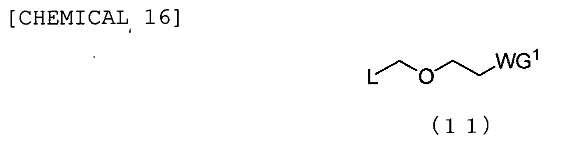 In the general formula (11), L represents halogen, an arylthio group, an alkylsulfoxide group or an alkylthio group. WG¹ represents an electron-withdrawing group.

11. The method for producing a phosphoramidite compound according to either of claims 9 and 10, wherein x is an integer in the range of 2 to 15.

12. The method for producing the phosphoramidite compound according to any one of claims 9 to 11, wherein a salt of the tertiary amine with hydrofluoric acid is triethylamine trihydrofluoride.

13. The method for producing the phosphoramidite compound according to any one of claims 9 to 12, wherein WG¹ is cyano.

14. The method for producing the phosphoramidite compound according to any one of claims 9 to 13, wherein the phosphoramiditing reagent is the compound represented by the following general formula (12a) or (12b). 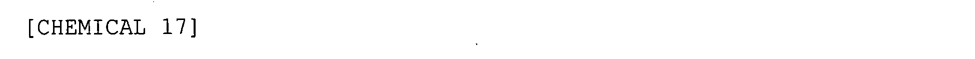 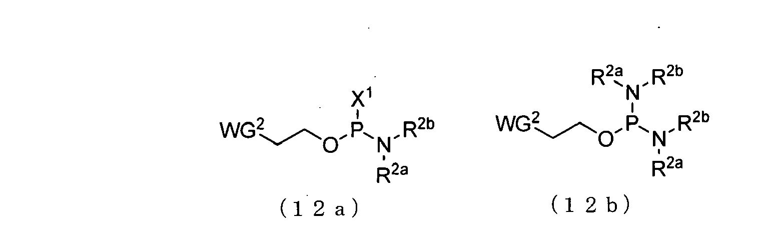 In the general formulae (12a) and (12b), R^{2a} and R^{2b} are the same or different and represent alkyl; or R^{2a} and R^{2b} represent a 5- or 6-membered saturated cyclic amino group when combined together with the adjacent nitrogen atom. The saturated cyclic amino group may have one oxygen atom or one sulfur atom as a ring-forming atom in addition to the nitrogen atom. WG² represents an electron-withdrawing group. X¹ represents halogen.

15. The method for producing the phosphoramidite compound according to any one of claims 9 to 14, wherein the activating reagent to use in Step f is 1H-tetrazole, 5-ethylthiotetrazole, 5-benzyl mercapto-1H-tetrazole, 4,5-dichloroimidazole, 4,5-dicyanoimidazole, benzotriazole triflate, imidazole triflate, pyridinium triflate, N,N-diisopropylethylamine or 2,4,6-collidine /N-methylimidazole.

16. A method for producing an oligoribonucleic acid represented by the following general formula (B), **characterized by** using a phosphoramidite compound produced by the method described in any one of claims 5 to 15. 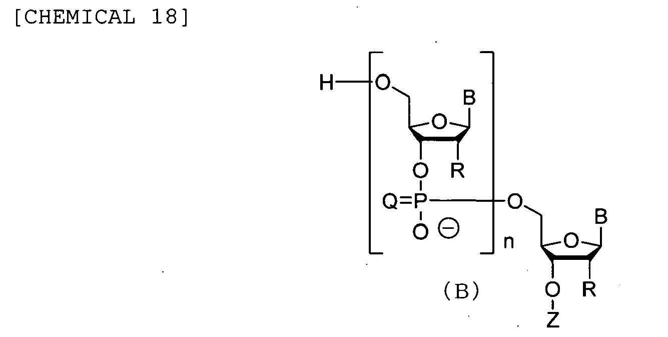 In the general formula (B), each B independently represents a nucleobase or a modified form thereof. Each Q independently represents O or S. Each R independently represents H, hydroxyl, halogen, alkoxy, alkylthio, alkylamino, dialkylamino, alkenyloxy, alkenylthio, alkenylamino, dialkenylamino, alkynyloxy, alkynylthio, alkynylamino, dialkynylamino or alkoxyalkyloxy, and at least one R is hydroxyl. Z represents H, a phosphate group or a thiophosphate group. n represents an integer in the range of 1 to 200.
